# EUROPEAN PATENT SPECIFICATION

(11) **EP 1 395 534 B1**
(45) Date of publication and mention of the grant of the patent: **30.08.2017**
(21) Application number: 02737127.7
(22) Date of filing: 24.05.2002
(51) Int. Cl.: C07C 67/475, C07C 69/145, C07C 67/297, C07C 67/293, C07C 6/04, C07D 207/20, C07D 303/04

(54) **IMPURITY INHIBITION IN OLEFIN METATHESIS REACTIONS**
VERMEIDUNG VON VERUNREINIGUNGEN IN OLEFIN-METATHESE
INHIBITION DES IMPURETES DANS LES REACTIONS DE METATHESE D'OLEFINES

(30) Priority: 24.05.2001 US 293931 P
(43) Date of publication of application: 10.03.2004
(73) Proprietor: Materia, Inc., Pasadena, CA 91107 (US)
(72) Inventor: Pederson Richard L, San Gabriel CA 91775 (US); Paulson Basil P., Vancouver, WA 98685 (CA)
(74) Representative: Barton, Matthew Thomas
(86) International application number: PCT/US2002/016372
(87) International publication number: WO 2002/094748

(56) References cited:
- US-A- 3 721 718
- US-A- 5 916 983
- SCHOLL M ET AL: "SYNTHESIS AND ACTIVITY OF A NEW GENERATION OF RUTHENIUM-BASED OLEFIN METATHESIS CATALYSTS COORDINATED WITH 1,3-DIMESITYL-4,5-DIHYDROIMIDAZ OL-2-YLIDENE LIGANDS" ORGANIC LETTERS, AMERICAN CHEMICAL SOCIETY, US, vol. 1, no. 6, 1999, pages 953-956, XP000984756 ISSN: 1523-7060 cited in the application

## Description

### BACKGROUND OF THE INVENTION

### Technical Field

The invention relates to metathesis catalytic reaction processes that reduce the production of unwanted byproducts and significantly improve yield, and thereby purity, of the desired reaction products. In particular, the invention relates to catalysts, inhibitors and reaction conditions for olefin metathesis reactions.

### Description of the Related Art

Over the past decade, olefin metathesis has emerged as a powerful carbon to carbon bond-forming reaction that is widely used in organic synthesis and polymer science (Trnka et al., Acc. Chem. Res. 34:18-29 (2001); Fürstner et al., Angew. Chem., Int. Ed. 39:3012-3043 (2000); Ivin et al., J. Mol. Catal. A: Chem. 133:1-16 (1998); Randall et al., J. Mol. Catal. A: Chem. 133:29-40 (1998); and Grubbs et al., Tetrahedron 54:4413-50 (1998)). In particular, ruthenium carbene olefin metathesis catalysts, and derivatives thereof, have firmly established olefin metathesis as a versatile and reliable synthetic technique for advanced organic synthesis. The exceptionally wide scope of substrates and functional group tolerance makes olefin metathesis a valuable technique that quickly and efficiently produces otherwise hard to make molecules, compared to traditional synthetic organic techniques. Research efforts have enabled the elucidation of olefin metathesis reactions catalyzed by various transition metal complexes. In particular, certain ruthenium and osmium carbene compounds, known as "Grubbs catalysts", have been identified as effective catalysts for olefin metathesis reactions such as, for example, cross-metathesis (CM), ring-closing metathesis (RCM), ring-opening metathesis (ROM), ring-opening metathesis polymerization (ROMP), or acyclic diene metathesis (ADMET) polymerization. The metathesis reaction products have a variety of uses, for example synthesized insect pheromone products may be used as selectively targeted pest control agents in agriculture. Accordingly, there is considerable interest in improved reaction product yields and purity.
US 5916983 discloses a method of synthesizing biologically active compounds. The compounds are produced by catalyzed cross metathesis of dissimilar terminal olefins or of a cyclodiene and a terminal olefin.

### SUMMARY OF THE INVENTION

The invention is defined by the claims. Any subject matter that falls outside of the scope of the claims is provided for information purposes only.

The present disclosure describes catalysts and reaction conditions to produce significantly improved yields (and thereby higher purity) of olefin metathesis products, with greatly reduced amounts of impurities. These techniques include in one instance reduction in temperature of reaction, and in another, the use of chemical compounds that act as reaction inhibitors of unwanted reactions to minimize or prevent formation of unwanted impurities.

The techniques of the disclosure are applicable to a wide range of metathesis reactions using a wide range of catalysts, including Grubbs, bis phosphine, and the like. The disclosure is useful in various cross-metathesis reactions, including but not limited to those that produce Peach Twig Borer pheromone, Omnivorous Leaf Roller pheromone. Tomato Pinworm pheromone, and 2-alkenal, acetates and alcohols. The disclosure is also useful in various ring-opening metathesis reactions, for example, to produce E-11-hexadecenyl acetate, Z-11-hexadecenyl acetate and Gypsy Moth pheromone; and in ring-closing various metathesis reactions, for example, to produce trifluoroacetyl protected N-3-pyrrolines.

Additional aspects and advantages of this disclosure will be apparent from the following detailed description of aspects thereof, which proceeds with reference to the accompanying drawings.

According to the present invention, there is provided an olefin metathesis reaction process comprising providing a reaction mixture comprising:
reactants comprising an olefin; and
an olefin metathesis catalyst comprising the general formula I or general formula II as shown below: 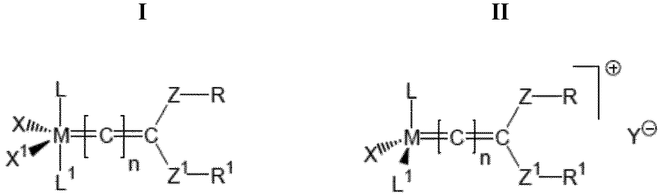 wherein:
   M is ruthenium or osmium;
   n is an integer between 0 and 5;
   L is a phosphine;
   L¹ is a nucleophilic carbene of the general formula III: 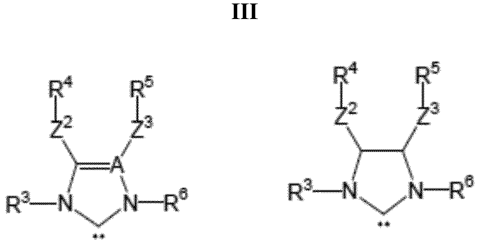 wherein:
      A is either carbon or nitrogen;
      R³, R⁴, R⁵, and R⁶ are each independently hydrogen or any hydrocarbyl moiety, except that in the case where A is nitrogen R⁵ is nil; and
      Z² and Z³ are each independently any linker selected from the group nil, -O-, -S-, -NR²-, - PR²-, -P(=O)R²-, -P(OR²)-, -P(=O)(OR²)-, -C(=O)-, -C(=O)O-, -OC(=O)-, -OC(=O)O-, -S(=O)-, or - S(=O)₂-, except that in the case where A is nitrogen Z³ is nil; and
      Z², Z³, R⁴, and R⁵ together optionally form a cyclic optionally substituted with one or more moieties selected from the group consisting of C₁-C₁₀ alkyl, C₁-C₁₀ alkoxy, aryl, and a functional group selected from the group consisting of hydroxyl, thiol, thioether, ketone, aldehyde, ester, ether, amine, imine, amide, nitro, carboxylic acid, disulfide, carbonate, isocyanate, carbodiimide, carboalkoxy, carbamate, and halogen;
      R, R¹ and R² are each independently hydrogen or any hydrocarbyl or silyl moiety;
      X and X¹ are each independently any anionic ligand;
      Y is any non coordinating anion; and
      Z and Z¹ are each independently any linker selected from the group: nil, -O-, -S-, -NR²-, -PR²-, -P(=O)R²-, -P(OR²)-, -P(=O)(OR²)-, -C(=O)-, -C(=O)O-, -OC(=O)-, -OC(=O)O-, -S(=O)-, or - S(=O)₂-; and
      the reaction process characterised in that the reaction mixture is maintained at a temperature in the range of from -72 °C to 14 °C when the olefin metathesis reaction producing the metathesis reaction product proceeds.

### 2. Preferably, M is ruthenium.

Advantageously, L¹ is selected from 1,3-dimesityl-4,5-dihydroimidazol-2-ylidene or 1,3-dimesityl-imidazol-ylidene.

Conveniently, L is selected from -P(cyclohexyl)₃, -P(cyclopentyl)₃, -P(isopropyl)₃, -P(butyl)₃, or - P(phenyl)₃; and L¹ is selected from: 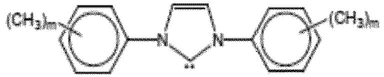 or 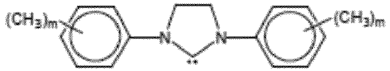 or 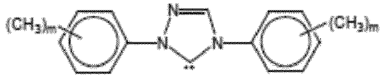 wherein m is an integer between 0 and 5.

Preferably, any two or more of X, X¹, L, L¹, Z, Z¹, R, and R¹ are joined together to form a multidentate ligand and/or wherein any one or more of X, X¹, L, L¹, Z, Z¹, R, and R¹ are linked chemically to a solid or glassy support.

Conveniently, any or all of L, L¹, R, and R¹ may be joined to form a bridging or cyclic structure.

Advantageously, any of the catalyst ligands include one of more functional groups selected from hydroxyl, thiol, thioether, ketone, aldehyde, ester, ether, amine, imine, amide, nitro, carboxylic acid, disulfide, carbonate, isocyanate, carbodiimide, carboalkoxy, carbamate, or halogen.

Preferably, the olefin metathesis catalyst is selected from (PCy₃)(s-IMes)Cl₂Ru=CH-CH=CMe₂, (PCp₃)(s-IMes)Cl₂Ru=CH-CH=CMe₂, (PPh₃)(s-IMes)Cl₂Ru=C=CHCMe₃, (PPh₃)(s-IMes)Cl₂Ru=C=CHSiMe₃,
(PPh₃)(s-IMes)Cl₂Ru=CH-CH=CMe₂, (PCy₃)(s-IMes)Cl₂Ru=C=CHPh, (PCp₃)(s-IMes)Cl₂Ru=C=CHPh, (PBu₃)(s-IMes) Cl₂Ru=C=CHPh, (PCy₃)(s-IMes)Cl₂Ru=CHPh, (PBu₃)(s-IMes)Cl₂Ru=CH-CH=CMe₂, (PCy₃)(IMes)Cl₂Ru=CH-CH=CMe₂, (PCp₃)(IMes)Cl₂Ru=CH-CH=CMe₂, (PPh₃)(IMes)Cl₂Ru=C=CHCMe₃, (PPh₃)(IMes)Cl₂Ru=C=CHSiMe₃, (PPh₃)(IMes)Cl₂Ru=CH-CH=CMe₂, (PCy₃)(IMes)Cl₂Ru=C=CHPh, (PCp₃)(IMes)Cl₂Ru=C=CHPh, (PBu₃)(IMes)Cl₂Ru=C=CHPh, (PCy₃)(IMes)Cl₂Ru=CHPh, (PBu₃)(IMes)Cl₂Ru=CH-CH=CMe₂, or (PCy₃)(IMes)Cl₂Ru=C=CHCMe₃.

Advantageously, the olefin metathesis catalyst is selected from ruthenium (II) [1,3-bis-(2,4,6-trimethylphenyl)-2-imidazolidinylidene) dichloro (3-methyl-1,2-butenylidene) (tricyclohexylphosphine), ruthenium (II) [1,3-bis-(2,4,6-trimethylphenyl)-2-imidazolidinyfidene) dichloro (phenylmethylene) (tricyclohexylphosphine) (metathesis catalyst 848), or ruthenium (II) [1,3-bis-(2,4,6-trimethylphenyl)-2-imidazolidinylidene) dichloro (phenylmethylene) (triphenylphosphine) (metathesis catalyst 830).

Preferably, the olefin metathesis catalyst comprises a Grubbs catalyst and the temperature is in the range from -5°C to 10°C.

Conveniently, the olefin metathesis catalyst comprises a Grubbs catalyst and the metathesis reaction is selected from ring opening reactions, wherein the percent yield of metathesis product is within 0 to 5% of the calculated theoretical yield.

Advantageously, R, R¹, or R² comprises a hydrocarbyl moiety selected from the group consisting of C₁-C₂₀ alkyl, C₃-C₂₀ cycloalkyl, C₂-C₂₀ alkenyl, C₂-C₂₀ alkynyl, aryl, heteroaryl, aralkyl, and alkaryl, or a silyl moiety selected from the group consisting of tri(hydrocarbyl)silyl, tri(hydrocarbyloxy)silyl, and mixed (hydrocarbyl)(hydrocarbyloxy)silyl; wherein each of R, R¹, or R² is optionally substituted with one or more hydrocarbyl or silyl moieties that are optionally further substituted with one or more groups selected from a halogen, a C₁-C₅ alkyl, C₁-C₅ alkoxy, or phenyl.

Conveniently, R is hydrogen and R¹ is selected from the group consisting of C₁-C₂₀ alkyl, C₂-C₂₀ alkenyl, aryl, alkaryl, aralkyl trialkylsilyl, and trialkoxysilyl.

Advantageously, n is 0, 1, or 2, and R¹ is phenyl, t-butyl, or vinyl, optionally substituted with one or more moieties selected from the group consisting of C₁-C₅ alkyl, C₁-C₅ alkoxy, phenyl, hydroxyl, thiol, thioether, ketone, aldehyde, ester, ether, amine, imine, amide, nitro, carboxylic acid, disulfide, carbonate, isocyanate, carbodiimide, carboalkoxy, carbamate, and halogen.

Preferably, n is 0 or 1, and R¹ is phenyl, t-butyl, or vinyl substituted with one or more moieties selected from chloride, bromide, iodide, fluoride, -NO₂, -NMe₂, methyl, methoxy, or phenyl.

Advantageously, X and X¹ are each independently hydrogen, halide, C₁-C₂₀ alkyl, aryl, C₁-C₂₀ alkoxide, aryloxide, C₃-C₂₀ alkyldiketonate, aryldiketonate, C₁-C₂₀ carboxylate, arylsulfonate, C₁-C₂₀ alkylsulfonate, C₁-C₂₀ alkylthiol, arylthiol, C₁-C₂₀ alkylsulfonyl, or C₁-C₂₀ alklysulfinyl; wherein X and X¹ are optionally substituted with one or more moieties selected from the group consisting of C₁-C₁₀ alkyl, C₁-C₁₀ alkoxy, and aryl, that are each optionally further substituted with one or more groups selected from halogen, C₁-C₅ alkyl, C₁-C₅ alkoxy, or phenyl.

Conveniently, X and X¹ are selected from halide, benzoate, C₁-C₅ carboxylate, C₁-C₅ alkyl, phenoxy, C₁-C₅ alkoxy, C₁-C₅ alkylthiol, arylthiol, aryl, or C₁-C₅ alkylsulfonate.

Advantageously, X and X¹ are selected from halide, CF₃CO₂, CH₃CO₂, CFH₂CO₂, (CH₃)₃CO, (CF₃)₂(CH₃)CO, (CF₃)(CH₃)₂CO, PhO, MeO, EtO. tosylate, mesylate, or trifluormethanesulfonate.

Preferably, X and X¹ are selected from chloride, bromide, or iodide.

Conveniently, the reactants comprise trans-1,10-diacetoxy-5-decene and trans-5-decene and the metathesis reaction product using a metathesis catalyst comprises trans and cis isomers of 5-decenyl acetate; wherein the reactants comprise cis-1,4-diacetoxy-2-butene and trans-5-decene and the metathesis reaction product using a Grubbs catalyst comprises trans and cis isomers of 2-heptenyl acetate; wherein the reactants comprise 1,2-epoxy-5-cyclooctene and trans-2-methyl-3-decene in the presence of H₂, 10% Pd/C and the metathesis reaction product using a Grubbs catalyst comprises 7,8-epoxy-2-methyl octadecane; or wherein the reactants comprise trans-1,8-diacetoxy-4-octene and trans-9-octadecene and the metathesis reaction product using a Grubbs catalyst comprises trans-4-tridecenyl acetate.

Preferably, the reaction employs a catalyst:olefin ratio between 1:5 and 11000000. Advantageously, a byproduct inhibitor is added to the olefin metathesis reaction mixture. Conveniently, the inhibitor is selected from the group consisting of quinone, halogenated quinones, quinone derivatives, butylated hydroxytolulene, vitamin E, halogenated alkanes, and halogenated aromatics.

Advantageously, the inhibitor is selected from the group consisting of electrophilic compounds, nucleophilic compounds, metal hydride inhibitors, and antioxidants.

Preferably, the olefin metathesis reaction occurs under neat conditions.

Conveniently, the olefin comprises 5-decene, the catalyst comprises a Grubbs catalyst, and the metathesis reaction product comprises 5-decenyl acetate.

Advantageously, the olefin comprises 1,4-diacetoxy-2-butene, the metathesis reaction product comprises 2-heptenyl acetate, and the olefin metathesis catalyst comprises a Grubbs catalyst. Preferably, the olefin metathesis catalyst comprises a Grubbs catalyst, wherein the reactants comprise 7-octenyl acetate, and the metathesis reaction product comprises Z-11-hexadecenyl acetate and/or E-11-hexadecenyl acetate.

Advantageously, the olefin metathesis catalyst comprises a Grubbs catalyst, the reactants comprise 2-methyl-3-decene and 1,2-epoxy-5-cyclooctene, and the metathesis reaction product is hydrogenated to form 7,8-epoxy-2-methyl octadecane.

Conveniently, the olefin metathesis reaction comprises a ring opening reaction, and the metathesis reaction product comprises a terminal olefin.

Advantageously, the metathesis reaction product comprises 4-tridecenyl acetate, a 2-alkenyl acetate, a 2-alkenyl alcohol, E-11-hexadecenyl acetate, or Z-11-hexadecenyl acetate.

Preferably, the olefin metathesis catalyst comprises a Grubbs catalyst and the olefin comprises 9-octadecene.

Conveniently, the metathesis reaction product comprises an insect pheromone.

### BRIEF DESCRIPTION OF THE DRAWINGS

The following drawings illustrate specific examples of aspects of the disclosure as an aid to explaining the disclosure and do not in any way limit the scope of the invention as claimed herein.

The invention is defined by the claims. Any subject matter that falls outside of the scope of the claims is provided for information purposes only.
FIGURE 1 is a depiction of a metathesis reaction;
FIGURE 2 is a depiction of a metathesis reaction;
FIGURE 3 is a depiction of a metathesis reaction;
FIGURE 4 is a depiction of a metathesis reaction;
FIGURE 5 is a depiction of a metathesis reaction;
FIGURE 6 is a depiction of a metathesis reaction;
FIGURE 7 is a depiction of a metathesis reaction;
FIGURE 8 is a depiction of a metathesis reaction;
FIGURE 9 is a depiction of a metathesis reaction;
FIGURE 10 is a depiction of formulae of certain metathesis catalysts;
FIGURE 11 is a graphical representation of analysis (by yield percent through gas chromatography, on the y-axis) versus time (x-axis) of a reaction mixture;
FIGURE 12 is a graphical representation of analysis (by yield percent through gas chromatography, on the y-axis) versus time (x-axis) of a reaction mixture; and
FIGURE 13 is a graphical representation of analysis (by yield percent through gas chromatography, on the y-axis) versus time (x-axis) of a reaction mixture.

### DETAILED DESCRIPTION OF PREFERRED EMBODIMENTS

The invention is defined by the claims. Any subject matter that falls outside of the scope of the claims is provided for information purposes only.

The present disclosure provides reaction conditions and chemical compound inhibitors that significantly improve product yields and purity in olefin metathesis reactions. The present disclosure may be applied to any suitable metathesis catalyst reaction to obtain yield enhancement and impurity reduction.

As an initial matter, "olefin metathesis," as it is understood in the art, refers to the metal-catalyzed redistribution of carbon-carbon bonds in a reaction involving an olefin. While the present disclosure is broadly applicable to almost all reactions involving olefin metathesis catalysts, some of these catalysts are better known than others. For example, over two decades of intensive research effort has culminated in the discovery of well-defined ruthenium and osmium carbenes that are highly active olefin metathesis catalysts and stable in the presence of a variety of functional groups. Among the catalysts of interest are the neutral ruthenium or osmium metal carbene complexes that possesses metal centers that are formally in the +2 oxidation state, have an electron count of 16, and are penta-coordinated. Other catalysts of particular interest include, but are not limited to, cationic ruthenium or osmium metal carbene complexes that possesses metal centers that are formally in the +2 oxidation state, have an electron count of 14, and are tetra-coordinated. Examples of such metathesis catalysts have been previously described in, for example, United States Patent Nos. 5,312,940; 5,342,909; 5,831,108; 5,969,170; 6,111,121; 6,211,391; 5,917,071; 5,977,393; and 6,225,488 and PCT Publications WO 98/39346, WO 99/00396, WO 99/00397, WO 99/28330, WO 99/29701, WO 99/50330, WO 99/51344, WO 00/15339, WO 00/58322 and WO 00/71554.

The ruthenium and osmium carbene complexes disclosed in these patents all possess metal centers that are formally in the +2 oxidation state, have an electron count of 16, and are penta-coordinated. These catalysts are of the general formula (I): 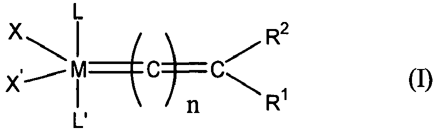 where n = 0 to 2, M is a Group 8 transition metal such as ruthenium or osmium, X and X' are anionic ligands, L and L' are neutral electron donors, and R and R' are specific substituents, e.g., one may be H and the other may be a substituted silyl, substituted, or unsubstituted hydrocarbyl group such as phenyl or -C=C(CH3)₂. Such complexes have been shown to be useful in catalyzing a variety of olefin metathesis reactions, including ring opening metathesis polymerization ("ROMP"), ring closing metathesis ("RCM"), acyclic diene metathesis polymerization ("ADMET"), ring-opening metathesis ("ROM"), and cross-metathesis ("CM" or "XMET") reactions. Their broad range of applications is due in large part to their excellent compatibility with various functional groups and relatively high tolerance to moisture, air, and other impurities (Schwab et al., Angew. Chem., Int. Ed. Engl. 34:2039-2041(1995); Schwab et al., J. Am. Chem. Soc. 118:100-110 (1996); Ivin, J. Mol. Cat. A-Chem. 133:1-16 (1998); Grubbs et al., Tetrahedron. 54:4413-4450 (1998); and Randall et al., J. Mol. Cat. A-Chem. 133, 29-40 (1998)). However, as has been recognized by those in the field, the compounds display low thermal stability, decomposing at relatively low temperatures. Jafarpour et al., Organometallics19(11):2055-2057 (2000).

For the most part, such metathesis catalysts have been prepared with phosphine ligands, e.g., tricyclohexylphosphine or tricyclopentylphosphine, exemplified by the well-defined, metathesis-active ruthenium alkylidene complexes (II) and (III): 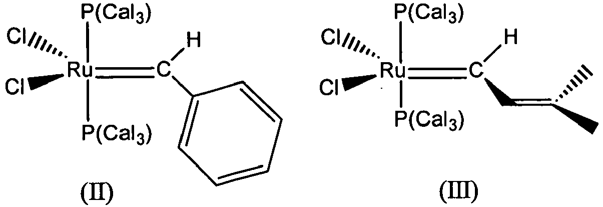 wherein "Cal" is a cycloalkyl group such as cyclohexyl or cyclopentyl. See Grubbs et al., U.S. Patent No. 5,917,071 and Trnka et al., supra. To increase the reactivity of ruthenium-based catalysts, replacement of one of the phosphine ligands with a 1,3-disubstituted-4,5-dihydro-(4,5-disubstituted)-imidazole-2-ylidene, such as 1,3-dimesityl-4,5-dihydroimidazol-2-ylidene, furnishes more active catalysts due to a more electron-rich ruthenium metal center (Scholl et al., Tetrahedron Letter 40:2247-2200 (1999) and Scholl et al., Org. Lett. 1-953-956 (1999)).

From these studies, it became apparent that the highly basic N-heterocyclic carbene ligands are an excellent ligand set for improvement in olefin metathesis reactivity, and may be superior alternatives to phosphines (Trnka et al., supra; Bourissou et al. Chem. Rev. 100:39-91 (2000); Scholl et al., Tet. Lett. 40:2247-2200 (1999); Scholl et al., Organic Lett. 1(6):953-956 (1999); and Huang et al., J. Am. Chem. Soc. 121:2674-2678 (1999)). N-heterocyclic carbene ligands offer many advantages, including readily tunable steric bulk, vastly increased electron donor character, and compatibility with a variety of metal species. In addition, replacement of one of the phosphine ligands in these complexes significantly improves thermal stability. The vast majority of research on these carbene ligands has focused on their generation and isolation, a feat finally accomplished by Arduengo and coworkers within the last ten years (see, e.g., Arduengo et al., Acc. Chem. Res. 32:913-921 (1999)). Four representative second generation catalysts are the ruthenium complexes (IVA), (IVB), (VA) and (VB): 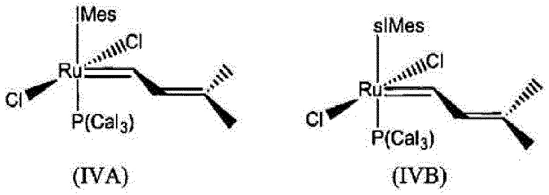 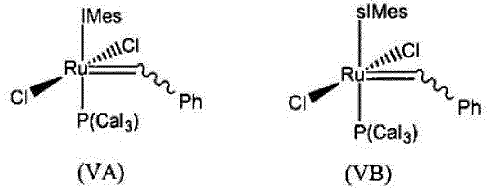 In the above structures, "Cal" is as defined previously, "Ph" represents phenyl, "IMes" represents 1,3-dimesityl-imidazol-2-ylidene: 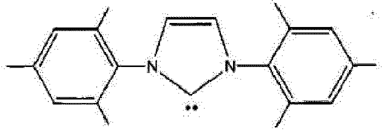 and "sIMes" represents 1,3-dimesityl-4,5-dihydroimidazol-2-ylidene: 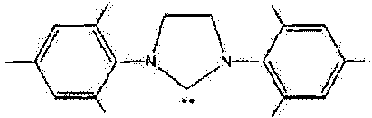 and "N-IMes" represents 1,3-dimesityl-triazolylidene;

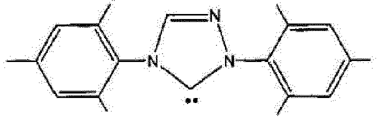

Other complexes formed from N-heterocyclic carbene ligands are also known. These transition metal carbene complexes, particularly those containing a ligand having the 4,5-dihydroimidazol-2-ylidene structure such as in sIMes, have been found to address a number of previously unsolved problems in olefin metathesis reactions, particularly cross-metathesis reactions.

All of the foregoing catalysts, and other olefin metathesis catalysts, may be used in accordance with the disclosure to obtain improved product yields and reduced impurity levels.

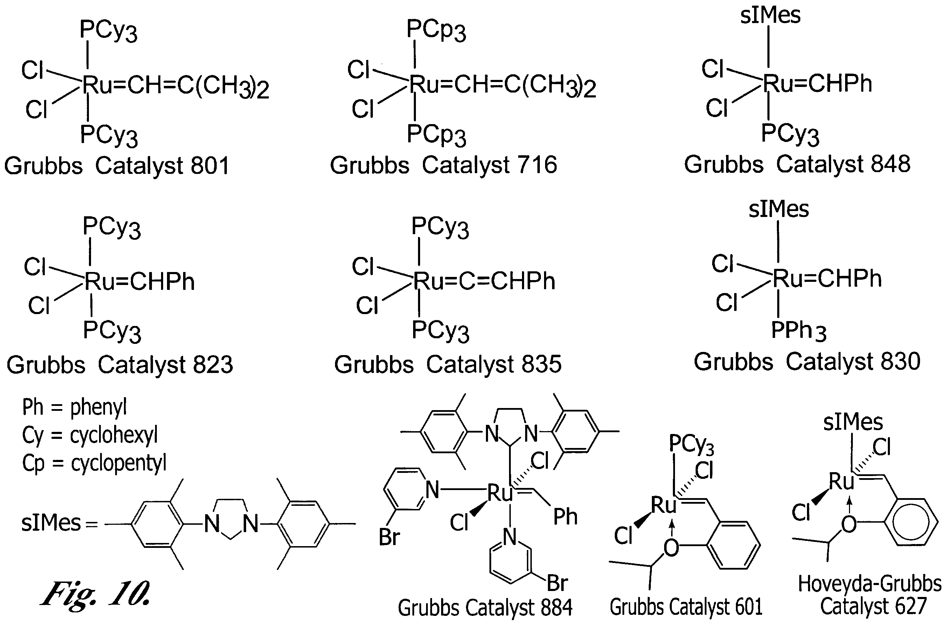

The prior art teaches that olefin metathesis catalysts must be heated for activation to initiate metathesis reactions. It has now been found that this approach generally renders the metathesis catalyst, including the second generation Grubbs catalysts (FIGURE 10. Catalysts 848, 830, 627, and 884), too aggressive in their activity. It is theorized, without being bound that upon heating the catalysts will migrate the double bonds of the starting materials and reactions products to produce undesired impurities, thereby lowering product yields. If left unchecked, the double bond migration reaction will quickly proceed resulting in a reaction product containing about 30% or more impurities, and yield reaction products that are not commercially viable due to low yield of useful product and purifying costs.

To overcome this shortcoming, the present disclosure, contrary to the prior art teaching, lowers the reaction temperature when an olefin metathesis catalyst is used. This results in some delay, of up to 7 hours, to initiate the metathesis reactions, but these reactions of the present disclosure do not produce significant amounts of impurities and produce very close to the theoretical conversion to desired product. In the specification and claims, unless otherwise indicated, the term "yield" refers to reaction product and the term "conversion" refers to reactants consumed in the reaction. In particular, percent conversion is calculated by determining the amount (mass or moles) of a reactant consumed as a percentage of the amount (mass or moles) of that reactant initially present before reaction commenced. Since many metathesis reactions are equilibrium reactions, that do not go to a final endpoint consuming all reactants, but can nonetheless be driven to consuming all of one reactant by adding an excess of another reactant, the theoretical or calculated yield of reaction product is dependant on the proportions of reactants, if more than one olefin is present. Thus, for a reaction consuming equal molar amounts of two reactants, and where equal molar amounts of reactants are present, the yield may be derived from the conversion of either of the reactants. For example, if one mole of each reactant is needed to produce one mole of product, and in fact 0.8 moles of each reactant was consumed out of 1.2 moles of each initially present (either due to equilibrium, or due to stopping of the reaction), and 0.6 moles of product was produced, then the percent yield is 100(0.6/0.8) = 75%. The percent conversion is 100(0.8/1.2) = 66.67%. In the event one of the reactants is present in excess amounts to drive the reaction beyond the conversion achieved at equimolar amounts of the reactants, then conversion and yield are based on the reactant that is more consumed (i.e. has lowest initial amount present). For example, assume reactants A and B combine in equimolar amounts to form C, and there are 5.0 moles of reactant A and 2.5 moles of reactant B present before reaction. At equilibrium, after reaction, or when the reaction is stopped, there are 3.0 moles of A remaining and 0.5 moles of B (i.e. 2.0 moles of A were consumed) and 1.8 moles of C. Percent conversion of A is 100(2.0/2.5) = 80%. Percent yield ofC is 100(1.8/2.0)= 90%. In a ring opening or ring closing reaction, where A converts to B, and one mole of A produces one mole of B. The percent conversion is 100 (moles of A consumed/moles of A present before reaction). The percent yield is 100 (moles of B produced/moles of A consumed). If no impurities are produced, the theoretical yield of 100% is achieved.

The disclosure provides yields that approach the theoretical yields quite closely. Thus, in accordance with the disclosure in yield is within 10%, preferably 0 to 5% of the theoretically calculated yield. For example, if theoretical yield is 40% based on reactants consumed, then actual yield is better than 30% (within 10%), and is preferably better than 35%(within 5%).

The metathesis reactions are run neat (i.e. without solvent) to maximize reactor space efficiency. Using an excess of one starting material will increase the yield of product but decrease the time throughput yield.

In general, in accordance with the disclosure, reaction is carried out at a temperature that will produce a high yield, where a "high yield is a yield of about 40% or more, when using equal molar ratios of starting materials or a yield that is about 80% or more of the theoretical maximum. Thus, for the N-heterocyclic carbene Grubbs catalysts, useful temperatures are in the range from -72°C to 14°C, and a temperature in the range about -5°C to about 10°C, is better, since it is more easily achievable and reaction rates are useful. A temperature of less than about 10°C is preferred, as is a temperature in the range about 5 to about 10°C.

In general, there is at least some inter relationship between temperature and yield: lower temperatures provide higher yields, but lowering temperature beyond a particular temperature (depending upon such factors as catalyst and reactants, for example) will at some point produce no further significant yield gain; i.e. there are diminishing yield gains. Further, a lower temperature reduces desired metathesis reaction rate as well, and requires longer batch reaction time. In addition, lower temperatures also delay reaction initiation (unless reaction is initiated at a higher temperature, and the reaction mixture is then cooled). So, there is a trade-off between (lower) reaction temperature, yield and reaction time.

In accordance with the disclosure, the temperature of a metathesis reaction using an s-IMes catalyst directly influences yield, and temperature reduction to about 0°C results in about 50% conversion to desired product, i.e. insignificant amounts of impurity (i.e. a total of less than about 3 to about 5%). Of this total, less than 0.1% is the double bond migrated impurity (for example, 4-decenyl acetate or 6-decenyl acetate in the case of catalysts 716, 801, 823 and 835 in Example 1, below). These positional isomers are very different to remove from 5-decenyl acetate because these materials are liquids and difficult to recrystallize and their boiling points are very similar. The bulk of the remaining impurities (i.e. about 2.5%) is the 4- or 5-nonenyl acetate and 5- and 6-undecenyl acetate, which can be removed by careful vacuum distillation. [It is suspected but not proven that starting materials may contain about 2% to 3% 4-decene or 1, 10-diacetoxy-4-decene, as impurities, which may account for the observed about 3% impurities.]

The bis phosphine Grubbs catalysts (FIGURE 10. Catalysts 801, 823, 716, and 601) are not as active as the s-IMes or IMes catalyst. These catalysts do not initiate appreciably at cold temperatures (e.g. less than about 10°C) and usually require the reaction mixture to be warmed to 30° to 75°C to obtain useful reaction rates. A disadvantage of warming the reaction mixture is that it increases the secondary reaction rate that produces impurities (these impurities are mainly double bond migrated species). In accordance with the disclosure, it has now been found that this (undesirable) secondary reaction rate is more sensitive to temperature than the metathesis reaction rate that produces desired product. In accordance with the disclosure, the secondary reaction rate is reduced to virtual insignificance by temperature reduction. In accordance with one aspect of the disclosure, bis phosphine catalyzed reaction mixtures are initially warmed to a range of temperatures that allow reaction initiation, and are then cooled to reduce the rates of reactions that produce byproducts that reduce product yield. Thus, in accordance with the disclosure, a bis phosphine catalyzed reaction mixture is optionally first heated to about 35°C or more to initiate the reaction and is then cooled to the temperature range from about 0°C to about 14°C, and better yet, to a temperature from about 5°C to about 14°C. Preferably, the reaction mixture is cooled to less than about 10 to about 14°C.

In one aspect, the disclosure, also provides chemical inhibitors of the reaction rates of the reactions that produce undesirable byproducts, also known as impurities. An impurity is regarded as present in an "insignificant" amount in accordance with the disclosure, if it is present in a small amount, and is relatively easily removed. When prior art metathesis reactions are carried out in a solvent free environment, for example not in a methylene chloride solution as is typically done, then there is typically the formation of a substantial amount of undesirable impurities such as double bond migrated impurities. These new compounds can undergo further metathesis reactions to produce a compound with 1-carbon less and 1-carbon more than the desired product. This process can repeat until an equilibrium mixture of impurities is obtained. According to one aspect of the disclosure, such impurities can be reduced or eliminated by adding an inhibitor selected from the electrophilic compounds, nucleophilic compounds and free radical scavengers (antioxidants). According to the disclosure, compounds that act as "metal hydride inhibitors", such as the halogenated alkanes and halogenated aromatics, for example, carbon tetrachloride, alpha, alpha dichlorotoluene, 1,2-dichloro ethane, 1,2-dibromoethane, bromoethane, bromopropane, bromobutane, etc., iodoethane, iodopropane, iodobutane, etc. are useful inhibitors of metathesis reactions that produce such byproducts. Other useful inhibitors include antioxidants such as quinone, similar compounds to quinone such as BHT (butylated hydroxytoluene), Vitamin E as well as halogenated quinones. Inhibitor dosage ranges from about 0.009 mol% to 5 mol%, typically 0.1 mol% based on the moles of reactant.

In general, the metathesis catalysts of most interest include, neutral ruthenium or osmium metal carbene complexes that possesses metal centers that are formally in the +2 oxidation state, have an electron count of 16, are penta-coordinated, and are of the general formula I, shown below. Other catalysts of major interest include, but are not limited to, cationic ruthenium or osmium metal carbene complexes that possesses metal centers that are formally in the +2 oxidation state, have an electron count of 14, are tetra-coordinated, and are of the general formula II. 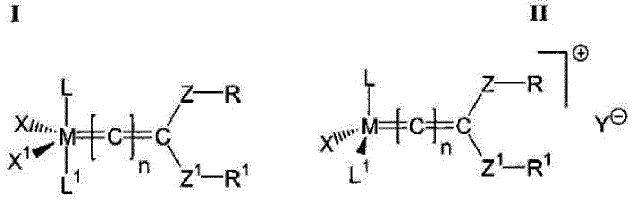 wherein:
M is ruthenium or osmium;
n is an integer between 0 and 5;
L is a phosphine and L¹ is a nucleophilic heterocyclic carbine of general formula III: 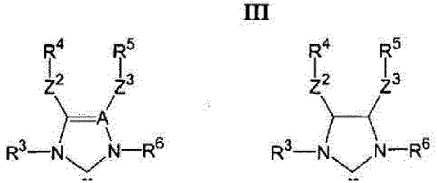
wherein:
A is either carbon or nitrogen;
R³, R⁴, R⁵, and R⁶ are each independently hydrogen or any hydrocarbyl moiety, except that in the case where A is nitrogen R⁵ is nil;
Z² and Z³ are each independently any linker selected from the group nil, -O-, -S-, -NR²-, -PR²-, -P(=O)R²-, -P(OR²)-, -P(=O)(OR²)-, -C(=O)-, -C(=O)O-, -OC(=O)-, -OC(=O)O-, -S(=O)-, or -S(=O)₂-, except that in the case where A is nitrogen Z³ is nil; and Z², Z³, R⁴, and R⁵ together may optionally form a cyclic optionally substituted with one or more moieties selected from the group consisting of C₁-C₁₀ alkyl, C₁-C₁₀ alkoxy, aryl, and a functional group selected from the group consisting of hydroxyl, thiol, thioether, ketone, aldehyde, ester, ether, amine, imine, amide, nitro, carboxylic acid, disulfide, carbonate, isocyanate, carbodiimide, carboalkoxy, carbamate, and halogen.
R, R¹, and R² are each independently hydrogen or any hydrocarbyl or silyl moiety;
X and X¹ are each independently any anionic ligand;
Y is any non coordinating anion;
Z and Z¹ are each independently any linker selected from the group nil, -O-, -S-, -NR², -PR²-, -P(=O)R²-, -P(OR²)-, -P(=O)(OR²)-, -C(=O)-, -C(=O)O-, -OC(=O)-, -OC(=O)O-, -S(=O)-, or -S(=O)₂-.

Although not in accordance with the present invention, in some catalysts of interest, L and L¹ are each a phosphine of the formula PR⁷R⁸R⁹, where R⁷, R⁸, and R⁹ are each independently any hydrocarbyl moiety, particularly aryl, primary C₁-C₁₀ alkyl, secondary alkyl or cycloalkyl. In certain other aspects, L and L¹ are selected from the group consisting of -P(cyclohexyl)₃, -P(cyclopentyl)₃, -P(isopropyl)₃, -P(butyl)₃, and -P(phenyl)₃.

According to the present invention, L is a phosphine and L¹ is a nucleophilic carbene of the general formula III. Preferably, L is selected from the group consisting of -P(cyclohexyl)₃, -P(cyclopentyl)₃, -P(isopropyl)₃, -P(butyl)₃, and -P(phenyl)₃ and L¹ is selected from the group consisting of: 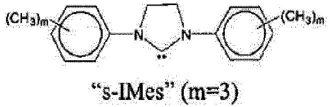 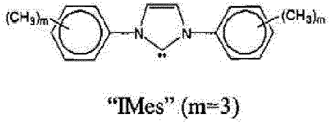 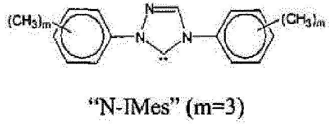 wherein m is an integer between 0 and 5.

Relating to R and R¹-R⁹, examples of hydrocarbyl moieties include, but are not limited to, the group consisting of C₁-C₂₀ alkyl, C₃-C₂₀ cycloalkyl, C₂-C₂₀ alkenyl, C₂-C₂₀ alkynyl, aryl, heteroaryl, aralkyl, or alkaryl. Examples of silyl moieties include, but are not limited to, tri(hydrocarbyl)silyl, tri(hydrocarbyloxy)silyl, or mixed (hydrocarbyl)(hydrocarbyloxy)silyl. Optionally, each of the R, R¹ or R² substituent groups may be substituted with one or more hydrocarbyl or silyl moieties, which, in turn, may each be further substituted with one or more groups selected from a halogen, a C₁-C₅ alkyl, C₁-C₅ alkoxy, and phenyl. Moreover, any of the catalyst ligands may further include one or more functional groups. Examples of suitable functional groups include but are not limited to: hydroxyl, thiol, thioether, ketone, aldehyde, ester, ether, amine, imine, amide, nitro, carboxylic acid, disulfide, carbonate, isocyanate, carbodiimide, carboalkoxy, carbamate, and halogen. In addition, any or all of R, R¹ and R² may be joined together to form a bridging or cyclic structure.

Also in addition, any or all of L, L¹, R, R¹ and R² may be joined to form a bridging or cyclic structure.

In aspects of interest, the R substituent is hydrogen and the R¹ substituent is selected from the group consisting C₁-C₂₀ alkyl, C₂-C₂₀ alkenyl, aryl, alkaryl, aralkyl, trialkylsilyl, and trialkoxysilyl. In certain preferred aspects, n equals 0, 1 or 2 and the R¹ substituent is phenyl, t-butyl or vinyl, optionally substituted with one or more moieties selected from the group consisting of C₁-C₅ alkyl, C₁-C₅ alkoxy, phenyl, and a functional group. In especially preferred aspects, n equals 0 or 1 and R¹ is phenyl, t-butyl, or vinyl substituted with one or more moieties selected from the group consisting of chloride, bromide, iodide, fluoride, -NO₂, -NMe₂, methyl, methoxy and phenyl.

In some aspects of interest, X and X¹ are each independently hydrogen, halide, or one of the following groups: C₁-C₂₀ alkyl, aryl, C₁-C₂₀ alkoxide, aryloxide, C₃-C₂₀ alkyldiketonate, aryldiketonate, C₁-C₂₀ carboxylate, arylsulfonate, C₁-C₂₀ alkylsulfonate, C₁-C₂₀ alkylthiol, aryl thiol, C₁-C₂₀ alkylsulfonyl, or C₁-C₂₀ alkylsulfinyl. Optionally, X and X¹ may be substituted with one or more moieties selected from the group consisting of C₁-C₂₀ alkyl, C₁-C₂₀ alkoxy, and aryl which in turn may each be further substituted with one or more groups selected from halogen, C₁-C₅ alkyl, C₁-C₅ alkoxy, and phenyl. In more preferred aspects, X and X¹ are halide, benzoate, C₁-C₅ carboxylate, C₁-C₅ alkyl, phenoxy, C₁-C₅ alkoxy, C₁-C₅ alkylthiol, aryl thiol, aryl, and C₁-C₅ alkyl sulfonate. In certain preferred aspects, X and X¹ are each halide, CF₃CO₂, CH₃CO₂, CFH₂CO₂, (CH₃)₃CO, (CF₃)₂(CH₃)CO, (CF₃)(CH₃)₂CO, PhO, MeO, EtO, tosylate, mesylate, or trifluoromethanesulfonate. In the most preferred aspects, X and X¹ are each chloride, bromide, or iodide. In addition, the X and X¹ together may comprise a bidentate ligand.

Y may be derived from any tetra coordinated boron compound or any hexa coordinated phosphorus compound. Preferred boron compounds include BF₄⁻, BPh₄⁻, and fluorinated derivatives of BPh₄⁻, but others are also useful. Preferred phosphorous compounds include, but are not limited to, PF₆⁻ and PO₄⁻². The non-coordinating anion may be also any one of the following: ClO₄⁻, SO₄⁼, NO₃⁻, OTeF₅⁻, F₃CSO₃⁻, H₃CSO₃⁻, CF₃COO⁻, PhSO₃⁻, or (CH₃)C₆H₅SO₃⁻. Y may be also derived from carboranes, chloro borates, carborane anions, fullerides, aluminoxanes.

The catalyst:olefin monomer ratio in the present disclosure is preferably between about 1:5 and about 1:1,000,000. More preferably, the catalyst:olefin ratio is about 1:1 to 1:200, or conforms with the literature, which usually puts the ratio in the range between about 1:10 and about 1:10,000 and, most preferably, between about 1:20 and about 1:1,000 or about 1:20 to 1:100. Particularly preferred metal catalysts include: (PCy₃)₂Cl₂Ru=CHPh,(PCy₃)₂Cl₂Ru=CH-CH=CMe₂, (PCy₃)₂Cl₂Ru=C=CHCMe₃, (PCy₃)₂Cl₂Ru=C=CHSiMe₃,(PCy₃)(s-IMes)Cl₂Ru=CH-CH=CMe₂, (PCp₃)₂Cl₂Ru=CH-CH=CMe₂, (PCp₃)₂Cl₂Ru=C-CHPh, (PCp₃)(s-IMes)Cl₂Ru=CH-CH=CMe₂, (PPh₃)(s-IMes)Cl₂Ru=C=CHCMe₃, (PPh₃)₂Cl₂Ru=C=CHSiMe₃, (PPh₃)₂Cl₂Ru=C=CHCMe₃, (P(i-Pr)₃)₂Cl₂Ru=C=CHPh, (PPh₃)(s-IMes]Cl₂Ru=C=CHSiMe₃, (PBu₃)₂Cl₂Ru=C=CHPh, (PPh₃)(s-IMes)Cl₂Ru=CH-CH=CMe₂, (PCy₃)(s-IMes)Cl₂Ru=C=CHPh, (PCp₃)(s-IMes)Cl₂Ru=C=CHPh, (PBu₃)(s-IMes)Cl₂Ru=C=CHPh, (PCy₃)(s-IMes)Cl₂Ru=CHPh, (PBu₃)(s-IMes)Cl₂Ru=CH-CH=CMe₂, (PCy₃)(IMes)Cl₂Ru=CH-CH=CMe₂, (PCp₃)IMes)Cl₂Ru=CH-CH=CMe₂, (PPh₃)(IMes)Cl₂Ru=C=CHCMe₃, (PPh₃)(IMes)Cl₂Ru=C=CHSiMe₃, (PPh₃)(IMes)Cl₂Ru=CH-CH=CMe₂, (PCy₃)(IMes)Cl₂Ru=C=CHPh, (PCp₃)(IMes)Cl₂Ru=C=CHPh, (PBu₃)(IMes)Cl₂Ru=C=CHPh, (PCy₃)(IMes)Cl₂Ru=CHPh, (PBu₃)(IMes)Cl₂Ru=CH-CH=CMe₂, (PCy₃)(IMes)Cl₂Ru=C=CHCMe₃, (PCy₃)ClRu=CHPh(o-O-Isop), (PCp₃)ClRu=CHPh(o-O-Isop), (PPh₃)ClRu=CHPh(o-O-Isop), (PBu₃)ClRu=CHPh(o-O-Isop), (s-IMes)ClRu=CHPh(o-O-Isop), (IMes)ClRu=CHPh(o-O-Isop), (N-s-IMes)ClRu=CHPh(o-O-Isop), and (N-IMes)ClRu=CHPh(o-O-Isop). Where (o-O-Isop) is ortho-isopropoxyphenyl methylene.

For convenience and reference herein, various examples of metathesis catalysts (shown in FIGURE 10) are identified by their molecular weight; ruthenium (II) dichloro (3-methyl-1,2-butenylidene) bis(tricyclopentylphosphine) (716); ruthenium (II) dichloro (3-methyl-1,2-butenylidene) bis(tricyclohexylphosphine) (801); ruthenium (II) dichloro (phenylmethylene) bis(tricyclohexylphosphine) (823); ruthenium (II) [1,3-bis-(2,4,6-trimethylphenyl)-2-imidazolidinylidene) dichloro (phenylmethylene) (tricyclohexylphosphine) (848); ruthenium (II) [1,3-bis-(2,4,6-trimethylphenyl)-2-imidazolidinylidene) dichloro (o-isopropoxyphenylmethylene) (627); ruthenium (II) [1,3-bis-(2,4,6-trimethylphenyl)-2-imidazolidinylidene) dichloro (phenylmethylene) (triphenylphosphine) (830), and ruthenium (II) dichloro (vinyl phenylmethylene) bis(tricyclohexylphosphine) (835); ruthenium (II) dichloro (tricyclohexylphosphine) (o-isopropoxyphenylmethylene) (601), and ruthenium (II) (1, 3-bis-(2,4, 6,-trimethylphenyl)-2-imidazolidinylidene) dichloro (phenylmethylene) (bis 3-bromopyridine (884)). This molecular weight-based nomenclature will be used the examples that follow.

The following examples merely serve to illustrate certain aspects of the disclosure for ease of explanation and are not to be construed as in any way limiting the scope of the present disclosure as described and claimed herein.

### Examples

In each of the Examples given herebelow, reference to "percent yield" of product should be interpreted as explained above. In general, when there are two reactants, since the metathesis reactions are equilibrium reactions, using equal molar ratios of symmetrical starting materials will produce a theoretical maximum of 50 % product yield, if half of the reactants are consumed. The consumed moles of reactants could also produce byproducts, also referred to as impurities herein. Thus actual yield is less than theoretical, but appraoches theoretical, in accordance with the disclosure.

The invention is defined by the claims. Any subject matter that falls outside of the scope of the claims is provided for information purposes only. For instance catalyst 801 with L = L1 used in the following examples is a comparative example.

### Example 1 : Screening Olefin Metathesis Catalysts for Activity and Impurity Profile

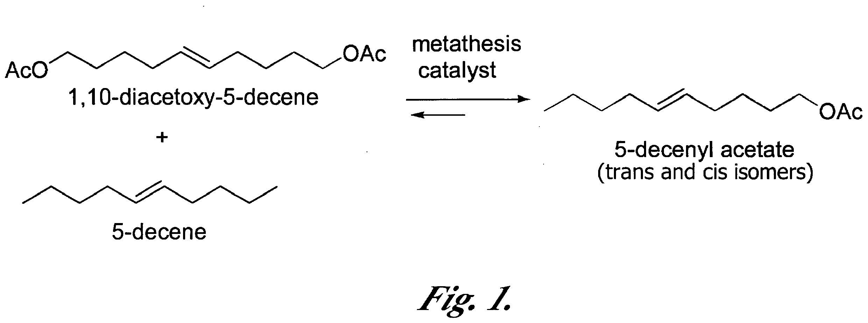

Grubbs olefin metathesis catalysts were screened for activity and impurity formation using the Argonaut Technologies (San Carlos, CA) FirstMate™ Manual Synthesizer. In the desired reaction, illustrated in FIGURE 1, equal moles of 5-decene and 1,10-diacetoxy-5-decene are reacted to produce 5-decenyl acetate. Six large test tubes (2.22 cm ID x 15.0 cm) were fitted to the FirstMate.™ Each test tube was filled with 1.67M 5-decene and 1.67M 1,10-diacetoxy-5-decene, in methylene chloride, to a final volume of 35 mL. The solutions were warmed to 45°C, under an inert gas, Argon. To each tube was charged one of the olefin metathesis catalysts 716, 801, 848, 823, 830, and 835 (0.117 mmol, 0.2 mol% catalyst), and mixing was initiated. Samples (∼1 mL) of the reaction mixture were withdraw from each tube, at times shown in Table 1, via a syringe and immediately quenched with 10 mL of 1 M trishydroxymethyl phosphine (THP in isopropyl alcohol (IPA), shaken for 1 min and placed in a 65°C oven for 1 hr. Water (5 mL) was added to each sample, shaken and the phases were allowed to separate. Three drops of the organic phase were added to separate 2 mL GC vials, diluted with IPA, and analyzed on a HP 5890 gas chromatograph (GC). Since the metathesis reactions are equilibrium reactions, using equal molar ratios of starting materials will produce a maximum of 50 % yield. Thus, percent conversion is based on moles of product divided by moles of both reactants consumed in the reaction. The consumed moles of reactants also would produce byproducts or impurities. The results of the Percent Conversion in each reaction tube (i.e. for each catalyst) are shown in Table 1. and Percent Impurities are shown in Table 2.

**Table 1. GC Percent Conversion to 5-Decenyl Acetate**

| Catalyst | Time | | | |
|---|---|---|---|---|
| | 5 min | 30 min | 6 hr | 20 hr |
| 716 | 1.2 | 2.8 | 9.6 | 9.7 |
| 801 | 3.5 | 11.3 | 30.2 | 29.5 |
| 823 | 4.5 | 11.1 | 22.9 | 21.7 |
| 848 | 46.3 | 42.8 | 31.7 | 31.2 |
| 830 | 43.7 | 43.0 | 34.2 | 30.8 |
| 835 | 0.5 | | 0.5 | 0.5 |

**Table 2. GC Percent Impurities Produced**

| Catalyst | Time | | | |
|---|---|---|---|---|
| | 5 min | 30 min | 6 hr | 20 hr |
| 716 | 4.0 | 4.5 | 8.5 | 5.0 |
| 801 | 3.5 | 4.6 | 9.6 | 6.4 |
| 823 | 3.5 | 4.4 | 7.4 | 4.1 |
| 848 | 9.0 | 16.1 | 37.6 | 37.9 |
| 830 | 13.9 | 16.0 | 30.5 | 33.4 |
| 835 | 5.8 | | 7.0 | 3.3 |

These results indicate that catalysts 848 and 830 produce the highest yields of cross-metathesis product after 5 minutes; however, they also produce the greatest amount of impurities, with greater than 30% impurities formed after 6 hr. We were encouraged by the rapid reaction rate and set out to inhibit impurity formation while still retaining a fast reaction rate and high conversion.

### Example 2: Cross-Metathesis Reaction with Catalyst 830 at -2°C

The experiment as described in Example 1 was reproduced with 35 mL of 1.67 M 5-decene and 1.67 M 1,10-diacetoxy-5-decene in methylene chloride, using only catalyst 830 (0.117 mmol, 0.2 mol% catalyst). The reaction was carried out at a colder temperature, -2°C, under Argon. The results of the percent conversion and percent impurities are shown in Table 3.

**Table 3. GC Results of Percent Conversion and Percent Impurities Produced using Catalyst 830 at -2°C.**

| Time | % Conversion | % Impurities | |
|---|---|---|---|
| | to 5-Decenyl Acetate | Produced | Detected |
| 0 min | 0.0 | 2.5* | 0 |
| 5 min | 0.8 | 4.7 | 0 |
| 15 min | 1.8 | 4.9 | 0 |
| 30 min | 20.5 | 6.9 | 0.8 |
| 1 hr | 43.5 | 7.3 | 1.7 |
| 2 hr | 46.6 | 7.5 | 1.8 |
| 8 hr | 44.90 | 11.1 | 2.4 |

| | | | |
|---|---|---|---|
| *At t=0 min, the sample was taken before Catalyst 830 was added. It has been observed that the metathesis catalyst removal technique introduces 2% to 4% impurity by GC. This impurity has been included in the Percent Impurties Detected column but subtracted out of the Impurities Produced column. | | | |

These results indicate that colder reaction temperatures slow the metathesis cross-coupling reaction but the reaction obtains close to theoretical conversion with 92% less impurities, compared to reactions run at elevated temperatures, see Table 2, 6 hour data point of 30.5% impurities compared with Table 3, 8 hour data point of 2.4% impurities.

### Example 3 : Cross-Metathesis Reaction with Metathesis Catalysts at 2 - 5°C

The experiment as described in Example 1 was reproduced with 35 mL of neat 10.93 g (78.0 mmol) 5-decene and 20.0 g (78.0 mmol) 1,10-diacetoxy-5-decene. This time the reaction mixtures contained 0.2 mol % each of catalyst 848, 830 and 801, and reactions were run between 2°C to 5°C, under Argon. Results of the percent conversion and percent impurities for catalysts 848, 830 and 801 are shown in Tables 4, 5 and 6 respectively.

**Table 4. GC Results of Percent Conversion and Percent Impurities Produced using Catalyst 848 at 2° to 5°C.**

| Time | % Conversion | % Impurities | |
|---|---|---|---|
| | to 5-Decenyl acetate | Produced | Detected |
| 0 min | 0.0 | 0.9 | 0 |
| 30 min | 0.0 | 3.3 | 0 |
| 1 hr | 0.0 | 4.5 | 0 |
| 3.5 hr | 1.2 | --- | --- |
| 7 hr | 20.8 | 3.2 | 0.8 |
| 12 hr | 43.9 | 4.6 | 2.0 |
| 24 hr | 48.9 | 5.4 | 1.9 |

**Table 5. GC Results of Percent Conversion and Percent Impurities Produced using Catalyst 830 at 2° to 5°C.**

| Time | %Conversion | % Impurities |
|---|---|---|
| | to 5-Decenyl acetate | Detected |
| 0 min | 0.0 | 0.9 |
| 30 min | 3.0 | --- |
| 1 hr | 4.0 | --- |
| 3.5 hr | 5.0 | --- |
| 7 hr | 6.0 | --- |
| 24 hr | 6.0 | --- |

**Table 6. GC Results of Percent Conversion and Percent Impurities Produced using Catalyst 801 at 2° to 5°C.**

| Time | % Conversion | % Impurities |
|---|---|---|
| | to 5-Decenyl acetate | Detected |
| 0 min | 0.0 | 0.9 |
| 30 min | 0.0 | --- |
| 1 hr | 0.0 | --- |
| 3.5 hr | 0.0 | --- |
| 7 hr | 0.5 | --- |
| 24 hr | 4.0 | --- |

These results indicate that catalyst 848 initiated at low temperatures, without solvents and few impurities are produced at these lower temperature. Comparing 1 hr impurities of 4.5% at 0% conversion and 24 hr impurities of 5.4% at 48.9% conversion, less than 1% new impurities were produced by the metathesis catalyst. Catalyst 830 did not initiate without the presence of an organic solvent and catalyst 801 did not initiate presumably because the reaction temperature was too cold.

### Example 4 : Determination of the Impurity Profile when Operated Under Extended Reaction Times

The following experiment determined the impurity profile after extended reaction times when run at -2°C. To a 20 mL round-bottomed flask was added 9 mL of neat 2.65 g (18.9 mmol) 5-decene and neat 4.8 g (18.9 mmol) 1,10-diacetoxy-5-decene, using (0.038 mmol, 0.2 mol%) catalyst 848, at -2°C under Argon. The results of the percent conversion and percent impurities are shown in Table 7.

**Table 7. GC Results of Percent Conversion and Percent Impurities Produced using Catalyst 848 at -2°C for extended time.**

| Time | 848 % Conversion | %Impurities | |
|---|---|---|---|
| | to 5-Decenyl acetate | Produced | Detected |
| 0 min | 0.0 | 0.9 | 0 |
| 6.75 hr | 3.0 | 1.6 | 0 |
| 18.15 hr | 38.8 | 3.4 | 0.9 |
| 27.15 hr | 46.0 | 4.8 | 0.9 |
| 41.0 hr | 47.3 | 5.4 | 1.6 |
| 61.5 hr | 48.1 | 4.8 | 1.7 |

These results indicate that running catalyst 848 under extended reaction times (i.e. 61.5 hr with 48.1 % conversion) does not generate significantly greater amounts of impurities as compared to the impurity level at the 27.15 hr data point, with 46.0% conversion.

### Example 5 : Synthesis of 2-Heptenyl Acetate: Cross Metathesis of 5-Decene and 1,4-Diacetoxy-2-Butene

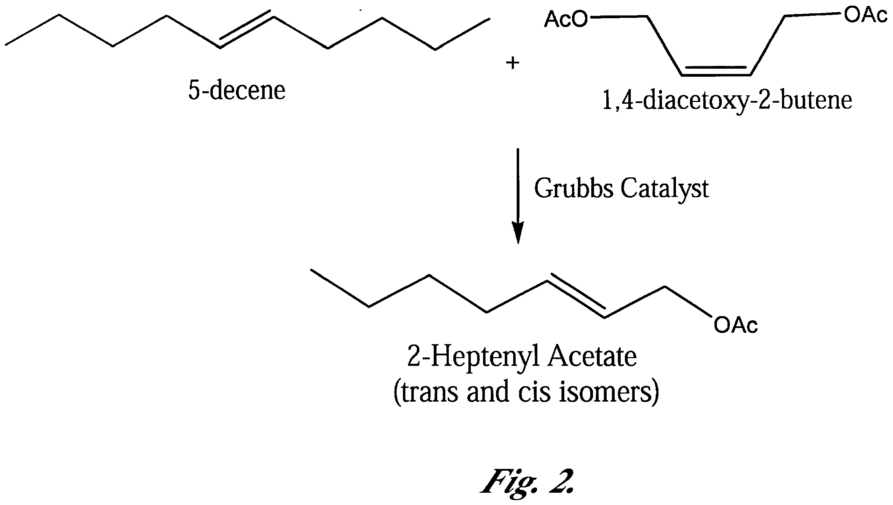

This experiment was designed to synthesize 2-heptenyl acetate in accordance with FIGURE 2. To a dry 50-mL round-bottomed flask was added 4 g (0.023 mol) 1,4-diacetoxy-2-butene, 3.3g (0.023 mol) 5-decene and a magnetic stir bar. The solution was cooled to 0°C and sparged with nitrogen for 10 minutes. Grubbs catalyst 848 (0.98 g, 1.2 mmol, 5 mol%) was added and the reaction was stirred at 0°C for 24 hours. The reaction was quenched by addition of 15 ml of 2M THP in IPA solution, warmed to 60°C with stirring for 24 hours. Water (5 mL) was added, stirred vigorously for 10 minutes, and phases separated. GC analysis of the organic phase indicated 25% 1,4-diacetoxy-2-butene, 25% 5-decene and 50% 2-heptenyl acetate; i.e., theoretical maximum yields were achieved.

### Example 6 : Synthesis of Z-11-Hexadecenyl Acetate: Cross Metathesis of 7-Octenyl Acetate and 1,2-Epoxy-5-Cyclooctene

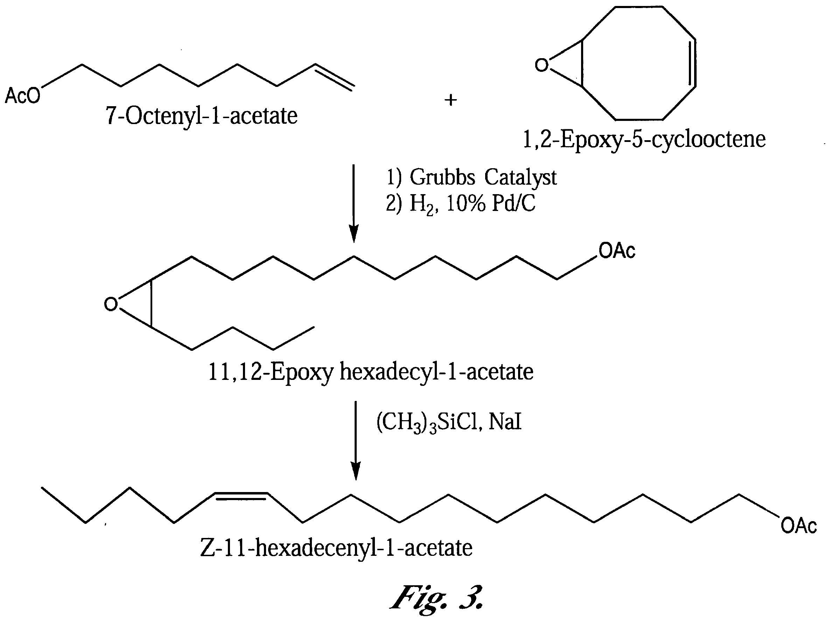

This example is designed to produce Z-11-hexadecenyl acetate through the reaction shown in FIGURE 3. To a dry 50-mL round-bottomed flask is added 4.5 g (0.026 mol) 7-octenyl acetate, 3.3g (0.026 mol) 1,2-epoxy-5-cyclooctene and a magnetic stir bar. The solution is cooled to 0°C and sparged with nitrogen for 10 minutes. Grubbs catalyst 848 (1.10 g, 1.30 mmol) is added and the reaction is stirred at 0°C for 24 hours. The reaction is quenched by addition of 15 ml of 2M THP in IPA solution, warmed to 60°C with stirring for 24 hours. Water (5 mL) is added and stirred vigorously for 10 minutes. The aqueous layer is separated, and the product washed with brine solution and dried over sodium sulfate. The product is purified by vacuum distillation, and then hydrogenated using 10% palladium on carbon (Pd/C) with a 5 psi overpressure of hydrogen. The catalyst is filtered off without further purification. The epoxide is converted to the cis olefin as described in (Tetrahedron Lett. 22, 3551 (1981)). There is sufficient detail to run the reaction 11,12-Epoxy-hexadecyl acetate is treated with 2.87 g (0.026 mol) chlorotrimethylsilane and 3.96 g (0.026 mol) sodium iodide. The reaction is slowly heated from room temperature to 60°C, until the starting materials are consumed. The mixture is then quenched with water and the organic phase was washed with an aqueous solution of sodium bicarbonate, and finally water. The organic phase is dried over sodium sulfate, acetylated with acetic anhydride and acetic acid and vacuum distilled to yield Z-11-hexadecenyl acetate.

### Example 7 : Synthesis of E-11-Hexadecenyl Acetate: Cross Metathesis of 7-Octenyl Acetate and 1,2-Epoxy-5-Cyclooctene

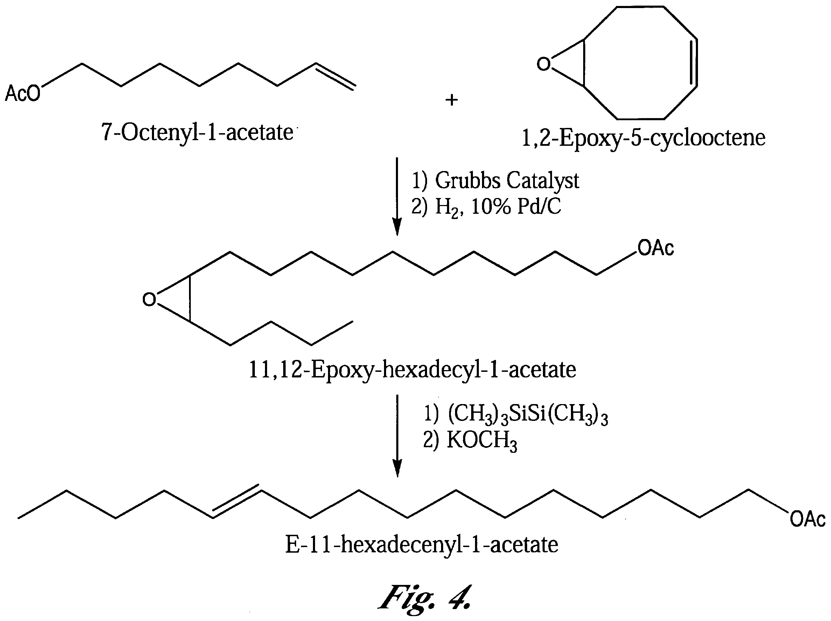

FIGURE 4 shows the reaction path for producing E-11-hexdecenyl acetate. To a dry 50-mL round-bottomed flask is added 4.5 g (0.026 mol) 7-octenyl acetate, 3.3g (0.026 mol) 1,2-epoxy-5-cyclooctene and a magnetic stir bar. The solution is cooled to 0°C and sparged with nitrogen for 10 minutes. Grubbs catalyst 848 (1.12 g, 1.31 mmol) is added and the reaction is stirred at 0°C for 24 hours. The reaction is quenched by addition of 15 ml of 2M THP in IPA solution, warmed to 60°C with stirring for 24 hours. Water (5 mL) is added and stirred vigorously for 10 minutes. The aqueous layer is separated, and the product washed with brine solution and dried over sodium sulfate. The product is purified by vacuum distillation, and then hydrogenated using 10% palladium on carbon with a 5 psi overpressure of hydrogen. The catalyst is filtered off without further purification. The epoxide is converted to the trans olefin as described in J. Amer. Chem. Soc. 98, 1265 (1976): J. Org. Chem. 41, 3063 (1976); Chem. Commun. 168 (1980)). 11,12-Epoxy-hexadecyl acetate is treated with 3.87 g (0.026 mol) hexamethyldisilane and 3.34 g (0.026 mol) potassium methoxide at room temperature (about 25°C). The solution is warmed to 50°C and stirred until the starting materials are consumed. The mixture is quenched with water and the organic phase is washed with 1 M hydrochloric acid, saturated aqueous solution of sodium bicarbonate, and finally brine. The organic phase is dried over sodium sulfate and vacuum distilled to yield E-11-hexadecenyl acetate

### Example 8 : Synthesis of Gypsy moth pheromone: 7,8-epoxy-2-methyl octadecane by Cross Metathesis of 2-Methyl-3-Decene and 1,2-Epoxy-5-Cyclooctene

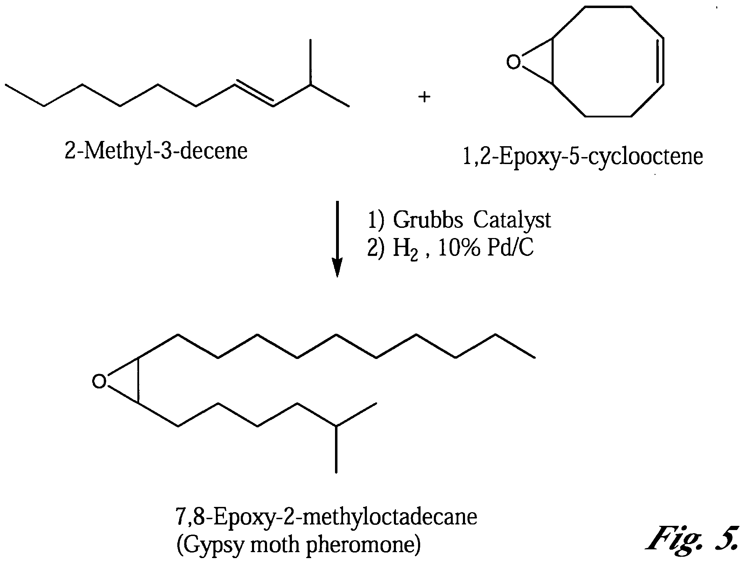

The reaction for this example is shown in FIGURE 5. To a dry 1-L round-bottomed flask is added 31.0 g (0.20 mol) 2-methyl-3-decene, 24.9g (0.20 mol) 1,2-epoxy-5-cyclooctene and a magnetic stir bar. The solution is cooled to 0°C and sparged with nitrogen for 10 minutes. Grubbs catalyst 848 8.5 g (1.0 mol) is added and the reaction was stirred at 0°C for 24 hours. The reaction is quenched with 20 ml of 2M THP in IPA, warmed to 60°C for 24 hours. The aqueous layer is separated, and the product washed with brine solution and dried over sodium sulfate. The product is purified by vacuum distillation, and then hydrogenated using 10% palladium on carbon with a 5 psi overpressure of hydrogen until the starting material is consumed. The catalyst is filtered off and the product is distilled under vacuum to yield the Gypsy moth pheromone

### Example 9 : Cross-Metathesis of 1-Butene and 11-Eicosenyl Acetate

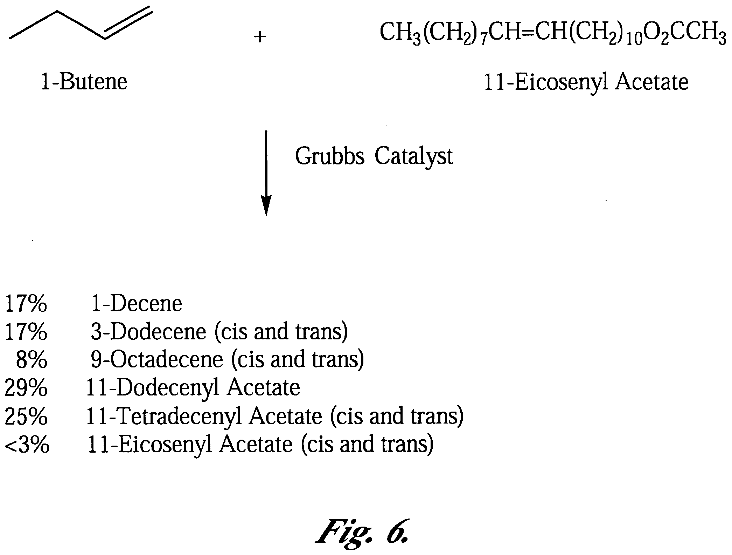

The reaction for this example is shown in FIGURE 6. To a dry 500-mL round-bottomed flask was added 50 g (0.15 mol) 11-eicosenyl acetate, and a magnetic stir bar. The vessel was cooled to 0°C and sparged with nitrogen for 10 minutes. Grubbs catalyst 848 (6.3g, 7.4 mmol) was added followed by 25g (0.44 mol) liquefied 1-butene. The reaction was stirred at 0°C for 24 hours. The reaction was quenched with 180 ml of 1M THP in IPA, warmed to room temperature 24°C, and stirred for 24 hours. The aqueous layer was separated, and the product washed with brine solution and dried over sodium sulfate. Without further purification, the product was analyzed by GCMS to identify the mixture of products as described below: The products are characterized by comparing peaks with known standards, in conjunction with supporting data from mass spectrum analysis using a mass spectrum detector (GCMS-Agilent 5973N). Column: DB-225 30m x 0.25mm (ID) x 0.25 µm film thickness. Manufacturer: J&W; GC and column conditions:
Injector temperature: 250° C
Oven temperature:
   Starting temperature: 40° C, hold time: 1 minute.
   Ramp rate 8° C/min to: 140° C, hold time: 5 minute.
   Ramp rate 20° C/min to: 210° C, hold time: 5 minute.
   Carrier gas: Helium
Mean gas velocity: 31.3 ± 3.5% cm/sec (calculated).
Split ratio: ∼50:1

These results demonstrate the catalyst 848 can catalyze disproportionation reactions to yield terminal olefin containing products.

### Example 10 : Inhibitor Studies of Impurities Formed in the Cross-Metathesis of 2-Pentene to yield 3-Hexene.

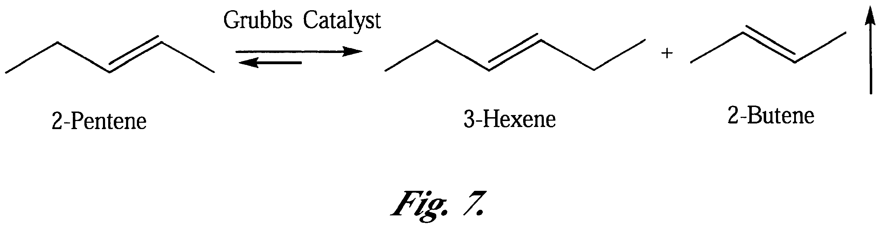

This example follows the reaction shown in FIGURE 7, 2-Pentene(16g, 230 mmol) was added to a dry 200 mL round-bottomed flask equipped with a condenser and a magnetic stir bar. The vessel was warmed to approximately 30°C using an oil bath and sparged with nitrogen for 5 minutes. Grubbs' Catalyst 848 (8.5 mg, 0.01 mmol) was then added to the vessel.

Aliquots of reaction mixture were taken at 15-minute increments and analyzed by GC. Samples were analyzed by diluting two drops of the reaction mixture in methylene chloride and analyzed by GC. GC analysis was performed on a HP5890 GC with FID detector, equipped with a capillary GC column, DB-220^{™} (30m x 25 mm ID x 25 um) column (GC method: 40°C for 1 min then 8°C/min to 118°CThe area percent of each peak or group of peaks were tabulated directly without correction for differences in response.

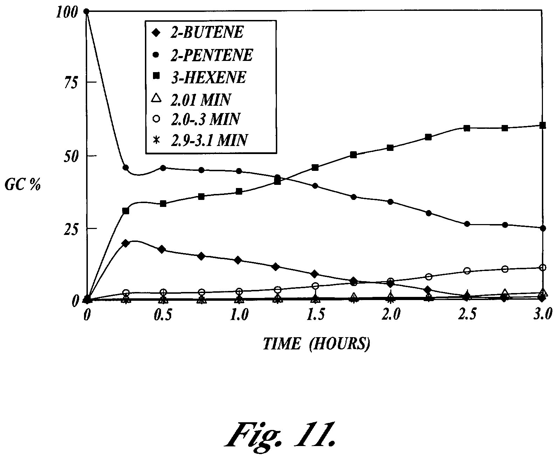

During these reactions, the temperature was raised slowly to remove 2-butene without loosing 2-pentene. The results are displayed in Figures 11, 12 and 13.

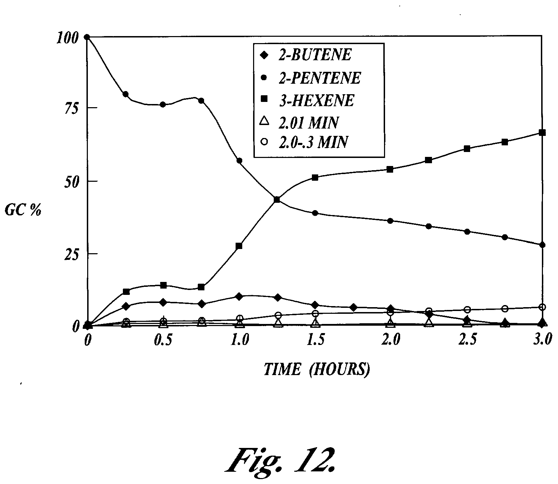

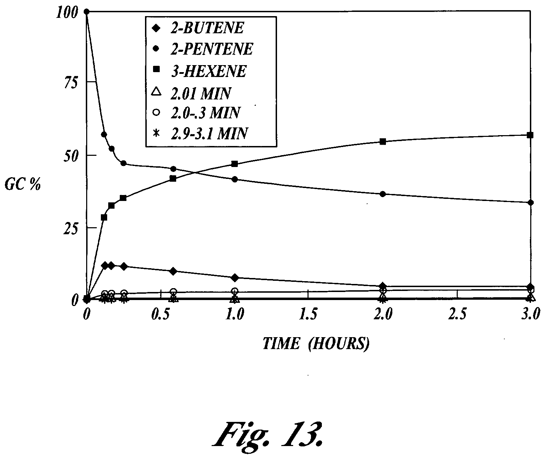

In the control experiment, Figure 11, the amount of impurities grew steadily to 14% of the reaction mixture after 3hr. Experiments with the addition of quinone (8.6 mol%) (FIGURE 12) and 1-bromo-3-chloropropane (5.0 mol%) (FIGURE 13) reduced the amount of impurities to 6% and 3%, respectively, while still obtaining about 60% product after three hours. These reactions demonstrate that 1-bromo-3-chloropropane, quinone and related compounds inhibit impurity formation and yield a commercially valuable product.

### Example 11 : Syntheses of Tomato Pinworm pheromone (4-Tridecenyl acetate): by Cross Metathesis of 1,8-Diacetaxy-4-Octene and 9-Octadecene

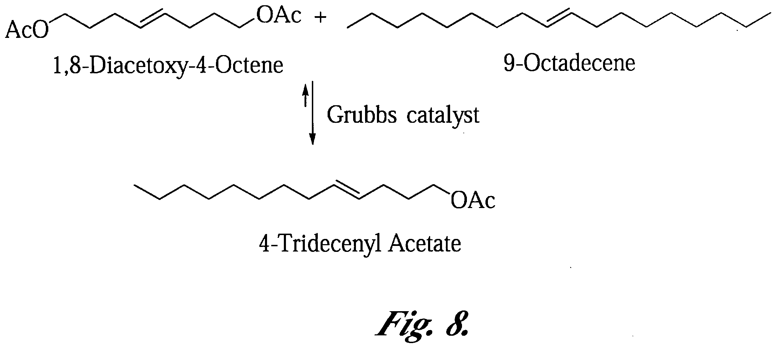

The reaction is shown in FIGURE 8. To a dry 500-mL round-bottomed flask was added 160.0 g (0.70 mol) 1,8-diacetoxy-4-octene, 88.0g (0.35 mol) 9-octadecene and a magnetic stir bar. The solution was cooled to 0°C and sparged with nitrogen for 20 minutes. Grubbs catalyst 848 1.48g (1.8 mol) was added and the reaction was stirred at 0°C for 24 hours. The reaction was quenched with 25 ml of 2M THP in IPA, warmed to 60°C for 12 hours. Water (100 mL was added and stirred vigorously for 30 minutes. The aqueous layer was separated, and the product washed with brine solution and dried over sodium sulfate. GC analysis of the reaction mixture indicated normalized values of 25% 1,8-diacetoxy-4-octene, 25% 9-octadecene and 50% 4-tridecenyl acetate.

### Example 12: Synthesis of TFA-Protected Pyrroline: by Ring-Closing Metathesis of N-TFA Diallylamine

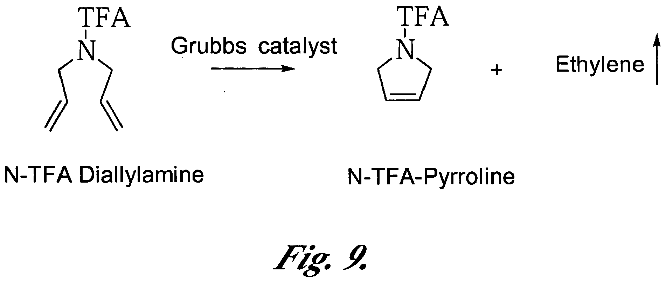

The reaction is shown in FIGURE 9. To a dry 500-mL round-bottomed flask was added 100.0 g (0.52 mol) of neat N-trifluoroacetyl (TFA) diallylamine and a magnetic stir bar. The solution was cooled to 10°C and sparged with nitrogen for 20 minutes. Grubbs catalyst 848] 4.4g (5.2 mmol) was added and the reaction was stirred at 0°C to 5°C, under 10 mmHg vacuum, for 18 hours. The reaction was quenched with 75 ml of 2M THP in IPA, warmed to 60°C for 12 hours. Water (100 mL) was added and stirred vigorously for 30 minutes. The aqueous layer was separated, and the product washed with brine solution and dried over sodium sulfate. GC analysis of the reaction mixture indicated 82% N-TFA pyrroline and 18% N-TFA diallylamine.

## Claims

1. An olefin metathesis reaction process comprising providing a reaction mixture comprising:
reactants comprising an olefin; and
an olefin metathesis catalyst comprising the general formula I or general formula II as shown below: 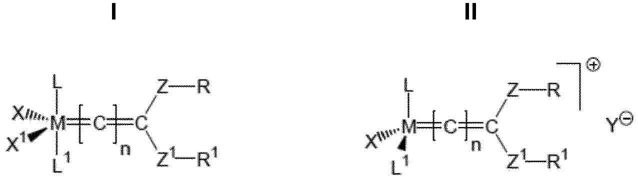 wherein:
M is ruthenium or osmium;
n is an integer between 0 and 5;
L is a phosphine;
L¹ is a nucleophilic carbene of the general formula III: 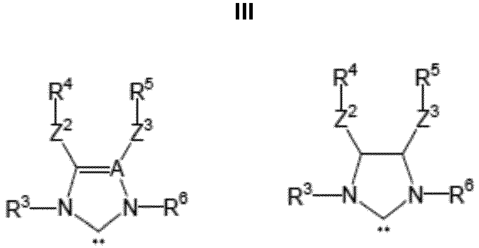 wherein:
A is either carbon or nitrogen;
R³, R⁴, R⁵, and R⁶ are each independently hydrogen or any hydrocarbyl moiety, except that in the case where A is nitrogen R⁵ is nil; and
Z² and Z³ are each independently any linker selected from the group nil, -O-, -S-, -NR²-, -PR²-, -P(=O)R²-, -P(OR²)-, -P(=O)(OR²)-, -C(=O)-, -C(=O)O-, -OC(=O)-, -OC(=O)O-, -S(=O)-, or - S(=O)₂-, except that in the case where A is nitrogen Z³ is nil; and
Z², Z³, R⁴, and R⁵ together optionally form a cyclic optionally substituted with one or more moieties selected from the group consisting of C₁-C₁₀ alkyl, C₁-C₁₀ alkoxy, aryl, and a functional group selected from the group consisting of hydroxyl, thiol, thioether, ketone, aldehyde, ester, ether, amine, imine, amide, nitro, carboxylic acid, disulfide, carbonate, isocyanate, carbodiimide, carboalkoxy, carbamate, and halogen;
R, R¹, and R² are each independently hydrogen or any hydrocarbyl or silyl moiety;
X and X¹ are each independently any anionic ligand;
Y is any non coordinating anion; and
Z and Z¹ are each independently any linker selected from the group: nil, -O-, -S-, -NR²-, -PR²-, -P(=O)R²-, -P(OR²)-, -P(=O)(OR²)-, -C(=O)-, -C(=O)O-, -OC(=O)-, -OC(=O)O-, -S(=O)-, or - S(=O)₂-; and
the reaction process **characterised in that** the reaction mixture is maintained at a temperature in the range of from -72 °C to 14 °C when the olefin metathesis reaction producing the metathesis reaction product proceeds.

2. The olefin metathesis reaction process of Claim 1, wherein M is ruthenium.

3. The olefin metathesis reaction process of Claim 1, wherein L¹ is selected from 1,3-dimesityl-4,5-dihydroimidazol-2-ylidene or 1,3-dimesityl-imidazol-ylidene.

4. The olefin metathesis reaction process of Claim 1, wherein L is selected from -P(cyclohexyl)₃, -P(cyclopentyl)₃, -P(isopropyl)₃, -P(butyl)₃, or -P(phenyl)₃; and L¹ is selected from: 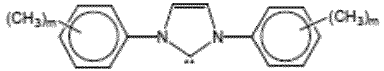 or 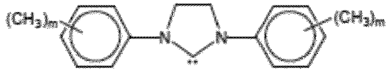 or 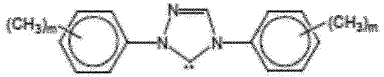 wherein m is an integer between 0 and 5.

5. The olefin metathesis reaction process of Claim 1, wherein any two or more of X, X¹, L, L¹, Z, Z¹, R, and R¹ are joined together to form a multidentate ligand and/or wherein any one or more of X, X¹, L, L¹, Z, Z¹, R, and R¹ are linked chemically to a solid or glassy support.

6. The olefin metathesis reaction process of Claim 1, wherein any or all of L, L¹, R, and R¹ may be joined to form a bridging or cyclic structure.

7. The olefin metathesis reaction process of Claim 1, wherein any of the catalyst ligands include one or more functional groups selected from hydroxyl, thiol, thioether, ketone, aldehyde, ester, ether, amine, imine, amide, nitro, carboxylic acid, disulfide, carbonate, isocyanate, carbodiimide, carboalkoxy, carbamate, or halogen.

8. The olefin metathesis reaction process of Claim 1, wherein the olefin metathesis catalyst is selected from (PCy₃)(s-IMes)Cl₂Ru=CH-CH=CMe₂, (PCp₃)(s-IMes)Cl₂Ru=CH-CH=CMe₂, (PPh₃)(s-IMes)Cl₂Ru=C=CHCMe₃, (PPh₃)(s-IMes)Cl₂Ru=C=CHSiMe₃, (PPh₃₎(s-IMes)Cl₂Ru=CH-CH=CMe₂, (PCy₃)(s-IMes)Cl₂Ru=C=CHPh, (PCp₃)(s-IMes)Cl₂Ru=C=CHPh, (PBu₃)(s-IMes) Cl₂Ru=C=CHPh, (PCy₃)(s-IMes)Cl₂Ru=CHPh, (PBu₃)(s-IMes)Cl₂Ru=CH-CH=CMe₂, (PCy₃)(IMes)Cl₂Ru=CH-CH=CMe₂, (PCp₃)(IMes)Cl₂Ru=CH-CH=CMe₂, (PPh₃)(IMes)Cl₂Ru=C=CHCMe₃, (PPh₃)(IMes)Cl₂Ru=C=CHSiMe₃, (PPh₃)(IMes)Cl₂Ru=CH-CH=CMe₂, (PCy₃)(IMes)Cl₂Ru=C=CHPh, (PCp₃)(IMes)Cl₂Ru=C=CHPh, (PBu₃)(IMes)Cl₂Ru=C=CHPh, (PCy₃)(IMes)Cl₂Ru=CHPh, (PBu₃)(IMes)Cl₂Ru=CH-CH=CMe₂, or (PCy₃)(IMes)Cl₂Ru=C=CHCMe₃.

9. The olefin metathesis reaction process of Claim 1, wherein the olefin metathesis catalyst is selected from ruthenium (II) [1,3-bis-(2,4,6-trimethylphenyl)-2-imidazolidinylidene) dichloro (3-methyl-1,2-butenylidene) (tricyclohexylphosphine), ruthenium (II) [1,3-bis-(2,4,6-trimethylphenyl)-2-imidazolidinylidene) dichloro (phenylmethylene) (tricyclohexylphosphine) (metathesis catalyst 848), or ruthenium (II) [1,3-bis-(2,4,6-trimethylphenyl)-2-imidazolidinylidene) dichloro (phenylmethylene) (triphenylphosphine) (metathesis catalyst 830).

10. The olefin metathesis reaction process of Claim 1, wherein the olefin metathesis catalyst comprises a Grubbs catalyst and the temperature is in the range from -5°C to 10°C.

11. The olefin metathesis reaction process of Claim 1, wherein the olefin metathesis catalyst comprises a Grubbs catalyst and the metathesis reaction is selected from ring opening reactions, wherein the percent yield of metathesis product is within 0 to 5% of the calculated theoretical yield.

12. The olefin metathesis reaction process of Claim 1, wherein R, R¹, or R² comprises a hydrocarbyl moiety selected from the group consisting of C₁-C₂₀ alkyl, C₃-C₂₀ cycloalkyl, C₂-C₂₀ alkenyl, C₂-C₂₀ alkynyl, aryl, heteroaryl, aralkyl, and alkaryl, or a silyl moiety selected from the group consisting of tri(hydrocarbyl)silyl, tri(hydrocarbyloxy)silyl, and mixed (hydrocarbyl)(hydrocarbyloxy)silyl; wherein each of R, R¹, or R² is optionally substituted with one or more hydrocarbyl or silyl moieties that are optionally further substituted with one or more groups selected from a halogen, a C₁-C₅ alkyl, C₁-C₅ alkoxy, or phenyl.

13. The olefin metathesis reaction process of Claim 1, wherein R is hydrogen and R¹ is selected from the group consisting of C₁-C₂₀ alkyl, C₂-C₂₀ alkenyl, aryl, alkaryl, aralkyl trialkylsilyl, and trialkoxysilyl.

14. The olefin metathesis reaction process of Claim 1, wherein n is 0, 1, or 2, and R¹ is phenyl, t-butyl, or vinyl, optionally substituted with one or more moieties selected from the group consisting of C₁-C₅ alkyl, C₁-C₅ alkoxy, phenyl, hydroxyl, thiol, thioether, ketone, aldehyde, ester, ether, amine, imine, amide, nitro, carboxylic acid, disulfide, carbonate, isocyanate, carbodiimide, carboalkoxy, carbamate, and halogen.

15. The olefin metathesis reaction process of Claim 1, wherein n is 0 or 1, and R¹ is phenyl, t-butyl, or vinyl substituted with one or more moieties selected from chloride, bromide, iodide, fluoride, -NO₂, -NMe₂, methyl, methoxy, or phenyl.

16. The olefin metathesis reaction process of Claim 1, wherein X and X¹ are each independently hydrogen, halide, C₁-C₂₀ alkyl, aryl, C₁-C₂₀ alkoxide, aryloxide, C₃-C₂₀ alkyldiketonate, aryldiketonate, C₁-C₂₀ carboxylate, arylsulfonate, C₁-C₂₀ alkylsulfonate, C₁-C₂₀ alkylthiol, arylthiol, C₁-C₂₀ alkylsulfonyl, or C₁-C₂₀ alklysulfinyl; wherein X and X¹ are optionally substituted with one or more moieties selected from the group consisting of C₁-C₁₀ alkyl, C₁-C₁₀ alkoxy, and aryl, that are each optionally further substituted with one or more groups selected from halogen, C₁-C₅ alkyl, C₁-C₅ alkoxy, or phenyl.

17. The olefin metathesis reaction process of Claim 1, wherein X and X¹ are selected from halide, benzoate, C₁-C₅ carboxylate, C₁-C₅ alkyl, phenoxy, C₁-C₅ alkoxy, C₁-C₅ alkylthiol, arylthiol, aryl, or C₁-C₅ alkylsulfonate.

18. The olefin metathesis reaction process of Claim 1, wherein X and X¹ are selected from halide, CF₃CO₂, CH₃CO₂, CFH₂CO₂, (CH₃)₃CO, (CF₃)₂(CH₃)CO, (CF₃)(CH₃)₂CO, PhO, MeO, EtO. tosylate, mesylate, or trifluormethanesulfonate.

19. The olefin metathesis reaction process of Claim 1, wherein X and X¹ are selected from chloride, bromide, or iodide.

20. The olefin metathesis reaction process of Claim 1, wherein the reactants comprise trans-1,10-diacetoxy-5-decene and trans-5-decene and the metathesis reaction product using a metathesis catalyst comprises trans and cis isomers of 5-decenyl acetate; wherein the reactants comprise cis-1,4-diacetoxy-2-butene and trans-5-decene and the metathesis reaction product using a Grubbs catalyst comprises trans and cis isomers of 2-heptenyl acetate; wherein the reactants comprise 1,2-epoxy-5-cyclooctene and trans-2-methyl-3-decene in the presence of H₂, 10% Pd/C and the metathesis reaction product using a Grubbs catalyst comprises 7,8-epoxy-2-methyl octadecane; or wherein the reactants comprise trans-1,8-diacetoxy-4-octene and trans-9-octadecene and the metathesis reaction product using a Grubbs catalyst comprises trans-4-tridecenyl acetate.

21. The olefin metathesis reaction process of Claim 1, wherein the reaction employs a catalyst:olefin ratio between 1:5 and 1:1000000.

22. The olefin metathesis reaction process of Claim 1, wherein a byproduct inhibitor is added to the olefin metathesis reaction mixture.

23. The reaction mixture of Claim 22, wherein the inhibitor is selected from the group consisting of quinone, halogenated quinones, quinone derivatives, butylated hydroxytolulene, vitamin E, halogenated alkanes, and halogenated aromatics.

24. The reaction mixture of Claim 22, wherein the inhibitor is selected from the group consisting of electrophilic compounds, nucleophilic compounds, metal hydride inhibitors, and antioxidants.

25. The reaction mixture of Claim 1, wherein the olefin metathesis reaction occurs under neat conditions.

26. The olefin metathesis reaction process of Claim 1, wherein the olefin comprises 5-decene, the catalyst comprises a Grubbs catalyst, and the metathesis reaction product comprises 5-decenyl acetate.

27. The olefin metathesis reaction process of Claim 1, wherein the olefin comprises 1,4-diacetoxy-2-butene, the metathesis reaction product comprises 2-heptenyl acetate, and the olefin metathesis catalyst comprises a Grubbs catalyst.

28. The olefin metathesis reaction process of Claim 1, wherein the olefin metathesis catalyst comprises a Grubbs catalyst, wherein the reactants comprise 7-octenyl acetate, and the metathesis reaction product comprises Z-11-hexadecenyl acetate and/or E-11-hexadecenyl acetate.

29. The olefin metathesis reaction process of Claim 1, wherein olefin metathesis catalyst comprises a Grubbs catalyst, the reactants comprise 2-methyl-3-decene and 1,2-epoxy-5-cyclooctene, and the metathesis reaction product is hydrogenated to form 7,8-epoxy-2-methyl octadecane.

30. The olefin metathesis reaction process of Claim 1, wherein the olefin metathesis reaction comprises a ring opening reaction, and the metathesis reaction product comprises a terminal olefin.

31. The olefin metathesis reaction process of Claim 1, wherein the metathesis reaction product comprises 4-tridecenyl acetate, a 2-alkenyl acetate, a 2-alkenyl alcohol, E-11-hexadecenyl acetate, or Z-11-hexadecenyl acetate.

32. The olefin metathesis reaction process of Claim 1, wherein the olefin metathesis catalyst comprises a Grubbs catalyst and the olefin comprises 9-octadecene.

33. The olefin metathesis reaction process of Claim 1, wherein the metathesis reaction product comprises an insect pheromone.

## Patentansprüche

1. Olefinmetathese-Reaktionsvorgang, der das Bereitstellen eines Reaktionsgemisches umfasst, umfassend:
Reaktante, die ein Olefin umfassen; und
einen Olefinmetathese-Katalysator, der die allgemeine Formel I oder die allgemeine Formel II unten umfasst: 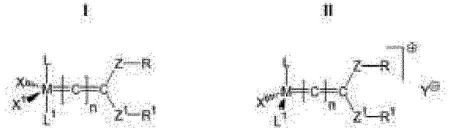 wobei:
M Ruthenium oder Osmium ist;
n eine ganze Zahl zwischen 0 und 5 ist;
L ein Phosphin ist;
L¹ ein nucleophiles Carben mit der allgemeinen Formel III ist: 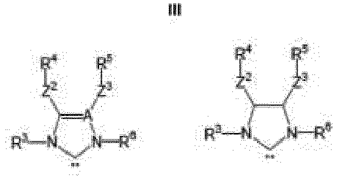 wobei:
A entweder Kohlenstoff oder Stickstoff ist;
R³, R⁴, R⁵ und R⁶ jeweils unabhängig voneinander Wasserstoff oder ein Kohlenwasserstoffrest sind, außer dass in dem Fall, in dem A Stickstoff ist, R⁵ entfällt; und
Z² und Z³ jeweils unabhängig voneinander beliebige Linker, ausgewählt aus der Gruppe entfällt, -O-, -S-, -NR²-, -PR²-, -P(=O)R²-, -P(OR²)-, -P(=O)(OR²)-, -C(=O)-, -C(=O)O-, - OC(=O)-, -OC(=O)O-, -S(=O)- oder -S(=O)₂- sind, außer dass in dem Fall, in dem A Stickstoff ist, Z³ entfällt; und
Z², Z³, R⁴ und R⁵ zusammen wahlweise eine zyklische Struktur bilden, wahlweise substituiert mit einem oder mehreren Resten bestehend aus C₁-C₁₀-Alkyl, C₁-C₁₀-Alkoxy, Aryl, und einer funktionellen Gruppe bestehend aus Hydroxy, Thiol, Thioether, Keton, Aldehyd, Ester, Ether, Amin, Imin, Amid, Nitro, Carbonsäure, Disulfid, Carbonat, Isocyanat, Carbodiimid, Carboalkoxy, Carbamat und Halogen;
R, R¹ und R² jeweils unabhängig voneinander Wasserstoff oder ein beliebiger Kohlenwasserstoffrest oder Silylrest sind;
X und X¹ jeweils unabhängig voneinander ein beliebiger anionischer Ligand sind;
Y ein beliebiges schwach koordinierendes Anion ist; und
Z und Z¹ jeweils unabhängig voneinander beliebige Linker, ausgewählt aus der Gruppe: entfällt, -O-, -S-, -NR²-, -PR²-, -P(=O)R²-, -P(OR²)-, -P(=O)(OR²)-, -C(=O)-, -C(=O)O-,-OC(=O)-, -OC(=O)O-, -S(=O)-, or -S(=O)₂- sind; und
der Reaktionsvorgang **dadurch gekennzeichnet ist, dass** das Reaktionsgemisch bei einer Temperatur im Bereich von -72 °C bis 14 °C gehalten wird, wenn die Olefinmetathese-Reaktion fortschreitet, die das Metathese-Reaktionsprodukt erzeugt.

2. Olefinmetathese-Reaktionsvorgang nach Anspruch 1, wobei M Ruthenium ist.

3. Olefinmetathese-Reaktionsvorgang nach Anspruch 1, wobei L¹ aus 1,3-Ddimesityl-4,5-dihydroimidazol-2-yliden oder 1,3-Dimesityl-imidazol-yliden ausgewählt ist.

4. Olefinmetathese-Reaktionsvorgang nach Anspruch 1, wobei L aus -P(cyclohexyl)₃, -P(cyclopentyl)₃, - P(isopropyl)₃, -P(butyl)₃ oder -P(phenyl)₃ ausgewählt ist; und L¹ ausgewählt ist aus: 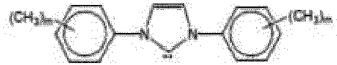 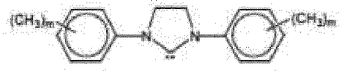 oder 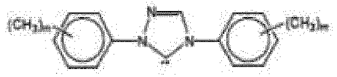 wobei m eine ganze Zahl zwischen 0 und 5 ist.

5. Olefinmetathese-Reaktionsvorgang nach Anspruch 1, wobei zwei oder mehrere beliebige aus X, X¹, L, L¹, Z, Z¹, R und R¹ miteinander verbunden sind, um einen mehrzähnigen Liganden zu bilden und/oder wobei ein beliebiger oder mehrere beliebige aus X, X¹, L, L¹, Z, Z¹, R und R¹ chemisch zu einem festen oder glasartigen Träger verbunden sind.

6. Olefinmetathese-Reaktionsvorgang nach Anspruch 1, wobei ein beliebiger oder alle aus L, L¹, R und R¹ miteinander verbunden sein können, um eine verbrückte oder eine zyklische Struktur zu bilden.

7. Olefinmetathese-Reaktionsvorgang nach Anspruch 1, wobei ein beliebiger der Katalysatorliganden eine oder mehrere funktionelle Gruppen ausgewählt aus Hydroxy, Thiol, Thioether, Keton, Aldehyd, Ester, Ether, Amin, Imin, Amid, Nitro, Carbonsäure, Disulfid, Carbonat, Isocyanat, Carbodiimid, Carboalkoxy, Carbamat oder Halogen umfasst.

8. Olefinmetathese-Reaktionsvorgang nach Anspruch 1, wobei der Olefinmetathese-Katalysator aus (PCy₃)(s-IMes)Cl₂Ru=CH-CH=CMe₂, (PCp₃)(s-IMes) Cl₂Ru=CH-CH=CMe₂, (PPh₃)(s-IMes)Cl₂Ru=C=CHCMe₃, (PPh₃)(s-IMes) Cl₂Ru=C=CHSiMe₃, (PPh₃)(s-IMes) Cl₂Ru=CH-CH=CMe₂, (PCy₃)(s-IMes) Cl₂Ru=C=CHPh, (PCp₃)(s-IMes)Cl₂Ru=C=CHPh, (PBu₃)(s-IMes)Cl₂Ru=C=CHPh, (PCy₃)(s-IMes) Cl₂Ru=CHPh, (PBu₃)(s-IMes) Cl₂Ru=CH-CH=CMe₂, (PCy₃)(IMes) Cl₂Ru=CH-CH=CMe₂, (PCp₃)(IMes) Cl₂Ru=CH-CH=CMe₂, (PPh₃)(IMes) Cl₂Ru=C=CHCMe₃, (PPh₃)(IMes) Cl₂Ru=C=CHSiMe₃, (PPh₃)(IMes) Cl₂Ru=CH-CH=CMe₂, (PCy₃)(IMes) Cl₂Ru=C=CHPh, (PCp₃)(IMes) Cl₂Ru=C=CHPh, (PBu₃)(IMes) Cl₂Ru=C=CHPh, (PCy₃)(IMes)Cl₂Ru=CHPh, (PBu₃)(IMes) Cl₂Ru=CH-CH=CMe₂ oder (PCy₃)(IMes)Cl₂Ru=C=CHCMe₃ ausgewählt ist.

9. Olefinmetathese-Reaktionsvorgang nach Anspruch 1, wobei der Olefinmetathese-Katalysator aus Ruthenium(II)[1,3-bis-(2,4,6-trimethylphenyl)-2-imidazolidinyliden)dichlor(3-methyl-1,2-butenyliden) (tricyclohexylphosphin), Ruthenium(II)[1,3-bis-(2,4,6-trimethylphenyl)-2-imidazolidinyliden)dichlor(phenylmethylen) (tricyclohexylphosp hin) (Metathesekatalysator 848) oder Ruthenium(II)[1,3-bis-(2,4,6-trimethylphenyl)-2-imidazolidinyliden)dichlor(phenylmethylen)(triphenylphosphin) (Metathesekatalysator 830) ausgewählt ist.

10. Olefinmetathese-Reaktionsvorgang nach Anspruch 1, wobei der Olefinmetathese-Katalysator einen Grubbs-Katalysator umfasst und die Temperatur im Bereich von -5 °C bis 10 °C liegt.

11. Olefinmetathese-Reaktionsvorgang nach Anspruch 1, wobei der Olefinmetathese-Katalysator einen Grubbs-Katalysator umfasst und die Metathese-Reaktion aus Ringöffnungsreaktionen ausgewählt ist, wobei der prozentuale Ertrag des Metathese-Produkts innerhalb von 0 bis 5 % des berechneten theoretischen Ertrags liegt.

12. Olefinmetathese-Reaktionsvorgang nach Anspruch 1, wobei R, R¹ oder R² einen Kohlenwasserstoffrest, ausgewählt aus der Gruppe bestehend aus C₁-C₂₀-Alkyl, C₃-C₂₀-Cycloalkyl, C₂-C₂₀-Alkenyl, C₂-C₂₀-Alkinyl, Aryl, Heteroaryl, Aralkyl und Alkaryl, oder einen Silylrest, ausgewählt aus der Gruppe bestehend aus Tri(hydrocarbyl)silyl, Tri(hydrocarbyloxy)silyl und gemischtem (Hydrocarbyl)(hydrocarbyloxy)silyl, umfassen; wobei jeder aus R, R¹ oder R² wahlweise mit einem oder mehreren Kohlenwasserstoffresten oder Silylresten substituiert ist, die wahlweise ferner mit einer oder mehreren Gruppen, ausgewählt aus einem Halogen, einem C₁-C₅-Alkyl, C₁-C₅-Alkoxy oder Phenyl, substituiert sind.

13. Olefinmetathese-Reaktionsvorgang nach Anspruch 1, wobei R Wasserstoff ist und R¹ aus der Gruppe bestehend aus C₁-C₂₀-Alkyl, C₂-C₂₀-Alkenyl, Aryl, Alkaryl, Aralkyltrialkylsilyl und Trialkoxysilyl ausgewählt ist.

14. Olefinmetathese-Reaktionsvorgang nach Anspruch 1, wobei n 0, 1 oder 2 ist und R¹ Phenyl, t-Butyl oder Vinyl ist, wahlweise substituiert mit einem oder mehreren Resten, ausgewählt aus der Gruppe bestehend aus C₁-C₅-Alkyl, C₁-C₅-Alkoxy, Phenyl, Hydroxy, Thiol, Thioether, Keton, Aldehyd, Ester, Ether, Amin, Imin, Amid, Nitro, Carbonsäure, Disulfid, Carbonat, Isocyanat, Carbodiimid, Carboalkoxy, Carbamat und Halogen.

15. Olefinmetathese-Reaktionsvorgang nach Anspruch 1, wobei n 0 oder 1 ist und R¹ Phenyl, t-Butyl oder Vinyl ist, substituiert mit einem oder mehreren Resten, ausgewählt aus Chlorid, Bromid, Iodid, Fluorid, -NO₂, -NMe₂, Methyl, Methoxy oder Phenyl.

16. Olefinmetathese-Reaktionsvorgang nach Anspruch 1, wobei X und X¹ jeweils unabhängig voneinander Wasserstoff, Halogenid, C₁-C₂₀-Alkyl, Aryl, C₁-C₂₀-Alkoxid, Aryloxid, C₃-C₂₀-Alkyldiketonat, Aryldiketonat, C₁-C₂₀-Carboxylat, Arylsulfonat, C₁-C₂₀-Alkylsulfonat, C₁-C₂₀-Alkylthiol, Arylthiol, C₁-C₂₀-Alkylsulfonyl oder C₁-C₂₀-Alklysulfinyl sind; wobei X und X¹ wahlweise mit einem oder mehreren Resten substituiert sind, ausgewählt aus der Gruppe bestehend aus C₁-C₁₀-Alkyl, C₁-C₁₀-Alkoxy und Aryl, die jeweils ferner mit einer oder mehreren Gruppen substituiert sind, ausgewählt aus Halogen, C₁-C₅-Alkyl, C₁-C₅-Alkoxy oder Phenyl.

17. Olefinmetathese-Reaktionsvorgang nach Anspruch 1, wobei X und X¹ aus Halogenid, Benzoat, C₁-C₅-Carboxylat, C₁-C₅-Alkyl, Phenoxy, C₁-C₅-Alkoxy, C₁-C₅-Alkylthiol, Arylthiol, Aryl oder C₁-C₅-Alkylsulfonat ausgewählt sind.

18. Olefinmetathese-Reaktionsvorgang nach Anspruch 1, wobei X und X¹ aus Halogenid, CF₃CO₂, CH₃CO₂, CFH₂CO₂, (CH₃)₃CO, (CF₃)₂(CH₃)CO, (CF₃) (CH₃)₂CO, PhO, MeO, EtO. Tosylat, Mesylat oder Trifluormethansulfonat ausgewählt sind.

19. Olefinmetathese-Reaktionsvorgang nach Anspruch 1, wobei X und X¹ aus Chlorid, Bromid oder Iodid ausgewählt sind.

20. Olefinmetathese-Reaktionsvorgang nach Anspruch 1, wobei die Reaktanten trans-1,10-Diacetoxy-5-decen und trans-5-Decen umfassen und das Metathese-Reaktionsprodukt, das einen Metathesekatalysator verwendet, trans- und cis-Isomere von 5-Decenylacetat umfasst; wobei die Reaktanten cis-1,4-Diacetoxy-2-buten und trans-5-Decen umfassen und das Metathese-Reaktionsprodukt, das einen Grubbs-Katalysator verwendet, trans- und cis-Isomere von 2-Heptenylacetat umfasst; wobei die Reaktanten 1,2-Epoxy-5-cycloocten und trans-2-Methyl-3-decen Gegenwart von H₂, 10 % Pd/C umfassen und das Metathese-Reaktionsprodukt, das einen Grubbs-Katalysator verwendet, 7,8-Epoxy-2-methyloctadecan umfasst; oder wobei die Reaktanten trans-1,8-Diacetoxy-4-octen und trans-9-Octadecen umfassen und das Metathese-Reaktionsprodukt, das einen Grubbs-Katalysator verwendet, trans-4-Tridecenylacetat umfasst.

21. Olefinmetathese-Reaktionsvorgang nach Anspruch 1, wobei für die Reaktion ein Katalysator-Olefin-Verhältnis zwischen 1:5 und 1:1000000 verwendet wird.

22. Olefinmetathese-Reaktionsvorgang nach Anspruch 1, wobei ein Nebenprodukthemmer zum Olefinmetathese-Reaktionsgemisch hinzugefügt wird.

23. Reaktionsgemisch nach Anspruch 22, wobei der Hemmer aus der Gruppe bestehend aus Chinon, halogenierten Chinonen, Chinonderivaten, Butylhydroxytoluol, Vitamin E, halogenierten Alkanen und halogenierten Aromaten ausgewählt ist.

24. Reaktionsgemisch nach Anspruch 22, wobei der Hemmer aus der Gruppe bestehend aus elektrophilen Verbindungen, nucleophilen Verbindungen, Metallhydridhemmern und Antioxidationsmitteln ausgewählt ist.

25. Reaktionsgemisch nach Anspruch 1, wobei bei die Olefinmetathese-Reaktion unter unverdünnten Bedingungen stattfindet.

26. Olefinmetathese-Reaktionsvorgang nach Anspruch 1, wobei das Olefin 5-Decen umfasst, der Katalysator einen Grubbs-Katalysator umfasst und das Metathese-Reaktionsprodukt 5-Decenylacetat umfasst.

27. Olefinmetathese-Reaktionsvorgang nach Anspruch 1, wobei das Olefin 1,4-Diacetoxy-2-buten umfasst, das Metathese-Reaktionsprodukt 2-Heptenylacetat umfasst und der Olefinmetathese-Katalysator einen Grubbs-Katalysator umfasst.

28. Olefinmetathese-Reaktionsvorgang nach Anspruch 1, wobei der Olefinmetathese-Katalysator einen Grubbs-Katalysator umfasst, wobei die Reaktanten 7-Octenylacetat umfassen und das Metathese-Reaktionsprodukt Z-11-Hexadecenylacetat und/oder E-11-Hexadecenylacetat umfasst.

29. Olefinmetathese-Reaktionsvorgang nach Anspruch 1, wobei der Olefinmetathese-Katalysator einen Grubbs-Katalysator umfasst, wobei die Reaktanten 2-Methyl-3-decen und 1,2-Epoxy-5-cycloocten umfassen, und das Metathese-Reaktionsprodukt hydriert wird, um 7,8-Epoxy-2-methyloctadecan zu bilden.

30. Olefinmetathese-Reaktionsvorgang nach Anspruch 1, wobei das Metathese-Reaktionsprodukt eine Ringöffnungsreaktion umfasst und das Metathese-Reaktionsprodukt ein terminales Olefin umfasst.

31. Olefinmetathese-Reaktionsvorgang nach Anspruch 1, wobei das Metathese-Reaktionsprodukt 4-Tridecenylacetat, ein 2-Alkenylacetat, ein 2-Alkenylalkohol, E-11-Hexadecenylacetat oder Z-11-Hexadecenylacetat umfasst.

32. Olefinmetathese-Reaktionsvorgang nach Anspruch 1, wobei der Olefinmetathese-Katalysator einen Grubbs-Katalysator umfasst und das Olefin 9-Octadecen umfasst.

33. Olefinmetathese-Reaktionsvorgang nach Anspruch 1, wobei das Metathese-Reaktionsprodukt ein Insektenpheromon umfasst.

## Revendications

1. Procédé de réaction de métathèse d'oléfines comprenant : fournir un mélange réactionnel comprenant :
des réactifs comprenant une oléfine ; et
un catalyseur de métathèse des oléfines comprenant la formule générale I ou la formule générale II telles que présentées ci-après : 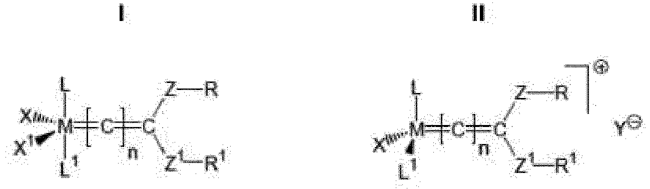 dans lesquelles :
M représente le ruthénium ou l'osmium ;
n est un entier entre 0 et 5 ;
L est une phosphine ;
L¹ est un carbène nucléophile de la formule générale III : 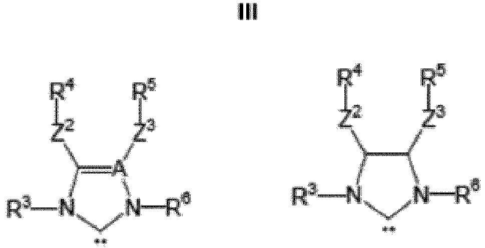 dans laquelle :
A représente soit le carbone soit l'azote ;
R³, R⁴, R⁵ et R⁶ représentent chacun indépendamment hydrogène ou toute fraction hydrocarbyle, excepté que, dans le cas où A représente l'azote, R⁵ est néant ; et
Z² et Z³ représentent chacun indépendamment tout lieur choisi dans le groupe : néant , -O-, -S-, -NR²-, - PR²-, -P(=O)R²-, -P(OR²)-, -P(=O)(OR²)-, -C(=O)-, -C(=O)O-, -OC(=O)-, -OC(=O)O-, -S(=0)- ou -S(=O)₂-, excepté que, dans le cas où A représente l'azote, Z³ est néant ; et
Z², Z³, R⁴ et R⁵ forment ensemble facultativement un groupe cyclique facultativement substitué par une ou plusieurs fractions choisies dans le groupe consistant en alkyle en C₁-C₁₀, alcoxy en C₁-C₁₀, aryle et un groupe fonctionnel choisi dans le groupe consistant en hydroxyle, thiol, thioéther, cétone, aldéhyde, ester, éther, amine, imine, amide, nitro, acide carboxylique, disulfure, carbonate, isocyanate, carbodiimide, carboalcoxy, carbamate et halogène ;
R, R¹ et R² représentent chacun indépendamment hydrogène ou toute fraction hydrocarbyle ou silyle ;
X et X¹ représentent chacun indépendamment tout ligand anionique ;
Y représente tout anion non coordinant ; et
Z et Z¹ représentent chacun indépendamment tout lieur choisi dans le groupe : néant, -O-, -S-, -NR²-, -PR²-, -P(=O)R²-, -P(OR₂)-, -P(=O)(OR²)-, -C(=O)-, -C(=O)O-, -OC(=O)-, -OC(=O)O-, -S(=O)- ou -S(=O)₂- ; et
le procédé de réaction étant **caractérisé par le fait que** le mélange réactionnel est maintenu à une température se situant dans la plage de -72°C à 14°C lorsque la réaction de métathèse des oléfines produisant le produit de réaction de métathèse se déroule.

2. Procédé de réaction de métathèse d'oléfines selon la revendication 1, dans lequel M représente le ruthénium.

3. Procédé de réaction de métathèse d'oléfines selon la revendication 1, dans lequel L¹ est choisi parmi le 1,3-dimésityl-4,5-dihydroimidazol-2-ylidène ou le 1,3-dimésityl-imidazol-ylidène.

4. Procédé de réaction de métathèse d'oléfines selon la revendication 1, dans lequel L est choisi parmi -P(cyclohexyle)₃, -P(cyclopentyle)₃, -P(isopropyle)₃, -P(butyle)₃ ou -P(phényle)₃ ; et L¹ est choisi parmi : 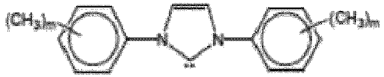 ou 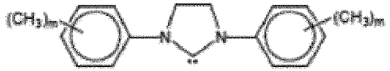 ou 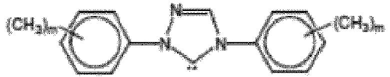 dans laquelle m est un entier entre 0 et 5.

5. Procédé de réaction de métathèse d'oléfines selon la revendication 1, dans lequel au moins deux quelconques parmi X, X¹, L, L¹, Z, Z¹, R et R¹ sont réunis ensemble pour former un ligand multidentate et/ou dans lequel au moins l'un quelconque parmi X, X¹, L, L¹, Z, Z¹, R et R¹ est lié chimiquement à un support solide ou vitreux.

6. Procédé de réaction de métathèse d'oléfines selon la revendication 1, dans lequel n'importe lesquels parmi ou tous les L, L¹, R et R¹ peuvent être réunis pour former une structure pontante ou cyclique.

7. Procédé de réaction de métathèse d'oléfines selon la revendication 1, dans lequel l'un quelconque des ligands de catalyseur comprend un ou plusieurs groupes fonctionnels choisis parmi hydroxyle, thiol, thioéther, cétone, aldéhyde, ester, éther, amine, imine, amide, nitro, acide carboxylique, disulfure, carbonate, isocyanate, carbodiimide, carboalcoxy, carbamate ou halogène.

8. Procédé de réaction de métathèse d'oléfines selon la revendication 1, dans lequel le catalyseur de métathèse d'oléfines est choisi parmi (PCy₃)(s-IMes)Cl₂Ru=CH-CH=CMe₂, (PCp₃)(s-IMes)Cl₂Ru=CH-CH=CMe₂, (PPh₃)(s-IMes)Cl₂Ru=C=CHCMe₃, (PPh₃)(s-IMes)Cl₂Ru=C=CHSiMe₃, (PPh₃)(s-IMes)Cl₂Ru=CH-CH=CMe₂, (PCy₃)(s-IMes)Cl₂Ru=C=CHPh, (PCp₃)(s-IMes)Cl₂Ru=C=CHPh, (PBu₃)(s-IMes)Cl₂ Ru=C=CHPh, (PCy₃)(s-IMes)Cl₂Ru=CHPh, (PBu₃)(s-IMes) Cl₂Ru=CH-CH=CMe₂, (PCy₃)(IMes)Cl₂Ru=CH-CH=CMe₂, (PCp₃)(IMes)Cl₂Ru=CH-CH=CMe₂, (PPh₃)(IMes)Cl₂Ru=C=CHCMe₃, (PPh₃)(IMes)Cl₂Ru=C=CHSiMe₃, (PPh₃)(IMes)Cl₂Ru=CH-CH=CMe₂, (PCy₃)(IMes)Cl₂Ru=C=CHPh, (PCp₃)(IMes)Cl₂Ru=C=CHPh, (PBu₃)(IMes)Cl₂Ru=C=CHPh, (PCy₃)(IMes)Cl₂Ru=CHPh, (PBu₃)(IMes)Cl₂Ru=CH-CH=CMe₂ ou (PCy₃)(IMes)Cl₂Ru=C=CHCMe₃.

9. Procédé de réaction de métathèse d'oléfines selon la revendication 1, dans lequel le catalyseur de métathèse d'oléfines est choisi parmi le ruthénium (II) [1,3-bis-(2,4,6-triméthylphényl)-2-imidazolidinylidène) dichloro (3-méthyl-1,2-buténylidène) (tricyclohexylphosphine), le ruthénium (II) [1,3-bis-(2,4,6-triméthylphényl)-2-imidazolidinylidène) dichloro (phénylméthylène) (tricyclohexylphosphine) (catalyseur de métathèse 848) ou le ruthénium (II) [1,3-bis-(2,4,6-triméthylphényl)-2-imidazolidinylidène) dichloro (phénylméthylène) (triphénylphosphine) (catalyseur de métathèse 830).

10. Procédé de réaction de métathèse d'oléfines selon la revendication 1, dans lequel le catalyseur de métathèse d'oléfines comprend un catalyseur de Grubbs et la température se situe dans la plage de -5°C à 10°C.

11. Procédé de réaction de métathèse d'oléfines selon la revendication 1, dans lequel le catalyseur de métathèse d'oléfines comprend un catalyseur de Grubbs et la réaction de métathèse est choisie parmi les réactions d'ouverture de cycle, le pourcentage de rendement du produit de métathèse se situant dans les 0 à 5 % du rendement théorique calculé.

12. Procédé de réaction de métathèse d'oléfines selon la revendication 1, dans lequel R, R¹ ou R² comprend une fraction hydrocarbyle choisie dans le groupe consistant en alkyle en C₁-C₂₀, cycloalkyle en C₃-C₂₀, alcényle en C₂-C₂₀, alcynyle en C₂-C₂₀, aryle, hétéroaryle, aralkyle et alcaryle, ou une fraction silyle choisie dans le groupe consistant en tri(hydrocarbyl)silyle, tri(hydrocarbyloxy)silyle et (hydrocarbyl)(hydrocarbyloxy)silyle mixte ; chacun parmi R, R¹ ou R² étant facultativement substitué par une ou plusieurs fractions hydrocarbyle ou silyle qui sont facultativement encore substituées par un ou plusieurs groupes choisis parmi un halogène, un alkyle en C₁-C₅, alcoxy en C₁-C₅ ou phényle.

13. Procédé de réaction de métathèse d'oléfines selon la revendication 1, dans lequel R représente hydrogène et R¹ est choisi dans le groupe consistant en alkyle en C₁-C₂₀, alcényle en C₂-C₂₀, aryle, alcaryle, aralkyle, trialkylsilyle et trialcoxysilyle.

14. Procédé de réaction de métathèse d'oléfines selon la revendication 1, dans lequel n est 0, 1 ou 2, et R¹ représente phényle, t-butyle ou vinyle, facultativement substitué par une ou plusieurs fractions choisies dans le groupe consistant en alkyle en C₁-C₅, alcoxy en C₁-C₅, phényle, hydroxyle, thiol, thioéther, cétone, aldéhyde, ester, éther, amine, imine, amide, nitro, acide carboxylique, disulfure, carbonate, isocyanate, carbodiimide, carboalcoxy, carbamate et halogène.

15. Procédé de réaction de métathèse d'oléfines selon la revendication 1, dans lequel n est 0 ou 1, et R¹ représente phényle, t-butyle ou vinyle substitué par une ou plusieurs fractions choisies parmi chlorure, bromure, iodure, fluorure, -NO₂, -NMe₂, méthyle, méthoxy ou phényle.

16. Procédé de réaction de métathèse d'oléfines selon la revendication 1, dans lequel X et X¹ représentent chacun indépendamment hydrogène, halogénure, alkyle en C₁-C₂₀, aryle, alcoxyde en C₁-C₂₀, aryloxyde, alkyldicétonate en C₃-C₂₀, aryldicétonate, carboxylate en C₁-C₂₀, arylsulfonate, alkylsulfonate en C₁-C₂₀, alkylthiol en C₁-C₂₀, arylthiol, alkylsulfonyl en C₁-C₂₀ ou alkylsulfinyle en C₁-C₂₀ ; X et X¹ étant facultativement substitués par une ou plusieurs fractions choisies dans le groupe consistant en alkyle en C₁-C₁₀, alcoxy en C₁-C₁₀ et aryle, qui sont chacun facultativement encore substitués par un ou plusieurs groupes choisis parmi halogène, alkyle en C₁-C₅, alcoxy en C₁-C₅ ou phényle.

17. Procédé de réaction de métathèse d'oléfines selon la revendication 1, dans lequel X et X¹ sont choisis parmi halogénure, benzoate, carboxylate en C₁-C₅, alkyle en C₁-C₅, phénoxy, alcoxy en C₁-C₅, alkylthiol en C₁-C₅, arylthiol, aryle ou alkylsulfonate en C₁-C₅.

18. Procédé de réaction de métathèse d'oléfines selon la revendication 1, dans lequel X et X¹ sont choisis parmi halogénure, CF₃CO₂, CH₃CO₂, CFH₂CO₂, (CH₃)₃CO, (CF₃)₂(CH₃)CO, (CF₃)(CH₃)₂CO, PhO, MeO, EtO. tosylate, mésylate ou trifluorométhanesulfonate.

19. Procédé de réaction de métathèse d'oléfines selon la revendication 1, dans lequel X et X¹ sont choisis parmi chlorure, bromure ou iodure.

20. Procédé de réaction de métathèse d'oléfines selon la revendication 1, dans lequel les réactifs comprennent le trans-1,10-diacétoxy-5-décène et le trans-5-décène et le produit de réaction de métathèse à l'aide d'un catalyseur de métathèse comprend les isomères trans et cis d'acétate de 5-décényle ; dans lequel les réactifs comprennent le cis-1,4-diacétoxy-2-butène et le trans-5-décène et le produit de réaction de métathèse à l'aide d'un catalyseur de Grubbs comprend les isomères trans et cis d'acétate de 2-heptényle ; dans lequel les réactifs comprennent le 1,2-époxy-5-cyclooctène et le trans-2-méthyl-3-décène en présence de H₂, Pd à 10 %/C et le produit de réaction de métathèse à l'aide d'un catalyseur de Grubbs comprend le 7,8-époxy-2-méthyl octadécane ; ou dans lequel les réactifs comprennent le trans-1,8-diacétoxy-4-octène et le trans-9-octadécène et le produit de réaction de métathèse à l'aide d'un catalyseur de Grubbs comprend l'acétate de trans-4-tridécényle.

21. Procédé de réaction de métathèse d'oléfines selon la revendication 1, dans lequel la réaction emploie un rapport catalyseur:oléfine entre 1:5 et 1:1 000 000.

22. Procédé de réaction de métathèse d'oléfines selon la revendication 1, dans lequel un inhibiteur de sous-produits est ajouté au mélange réactionnel de métathèse d'une oléfine.

23. Mélange réactionnel selon la revendication 22, dans lequel l'inhibiteur est choisi dans le groupe consistant en une quinone, les quinones halogénées, les dérivés de quinone, l'hydroxytolulène butylé, la vitamine E, les alcanes halogénés et les aromatiques halogénés.

24. Mélange réactionnel selon la revendication 22, dans lequel l'inhibiteur est choisi dans le groupe consistant en les composés électrophiles, les composés nucléophiles, les inhibiteurs d'hydrures métalliques et les anti-oxydants.

25. Mélange réactionnel selon la revendication 1, dans lequel la réaction de métathèse d'oléfines a lieu à l'état non dilué.

26. Procédé de réaction de métathèse d'oléfines selon la revendication 1, dans lequel l'oléfine comprend le 5-décène, le catalyseur comprend un catalyseur de Grubbs, et le produit de réaction de métathèse comprend l'acétate de 5-décényle.

27. Procédé de réaction de métathèse d'oléfines selon la revendication 1, dans lequel l'oléfine comprend le 1,4-diacétoxy-2-butène, le produit de réaction de métathèse comprend l'acétate de 2-heptényle et le catalyseur de métathèse d'oléfines comprend un catalyseur de Grubbs.

28. Procédé de réaction de métathèse d'oléfines selon la revendication 1, dans lequel le catalyseur de métathèse d'oléfines comprend un catalyseur de Grubbs, dans lequel les réactifs comprennent l'acétate de 7-octényle et le produit de réaction de métathèse comprend l'acétate de Z-11-hexadécényle et/ou l'acétate de E-11-hexadécényle.

29. Procédé de réaction de métathèse d'oléfines selon la revendication 1, dans lequel le catalyseur de métathèse d'oléfines comprend un catalyseur de Grubbs, les réactifs comprennent le 2-méthyl-3-décène et le 1,2-époxy-5-cyclooctène, et le produit de réaction de métathèse est hydrogéné pour former le 7,8-époxy-2-méthyl octadécane.

30. Procédé de réaction de métathèse d'oléfines selon la revendication 1, dans lequel la réaction de métathèse d'oléfines comprend une réaction d'ouverture de cycle, et le produit de réaction de métathèse comprend une oléfine terminale.

31. Procédé de réaction de métathèse d'oléfines selon la revendication 1, dans lequel le produit de réaction de métathèse comprend l'acétate de 4-tridécényle, un acétate de 2-alcényle, un 2-alcényl alcool, l'acétate de E-11-hexadécényle ou l'acétate de Z-11-hexadécényle.

32. Procédé de réaction de métathèse d'oléfines selon la revendication 1, dans lequel le catalyseur de métathèse d'oléfines comprend un catalyseur de Grubbs et l'oléfine comprend le 9-octadécène.

33. Procédé de réaction de métathèse d'oléfines selon la revendication 1, dans lequel le produit de réaction de métathèse comprend une phéromone d'insecte.
